(19) Europäisches Patentamt
European Patent Office
Office européen des brevets

(11) EP 1 841 737 B1

(12) **FASCICULE DE BREVET EUROPEEN**

(45) Date de publication et mention de la délivrance du brevet:
**10.03.2010 Bulletin 2010/10**

(21) Numéro de dépôt: **06704665.6**

(22) Date de dépôt: **04.01.2006**

(51) Int Cl.:
*C07D 209/42* (2006.01) *C07D 401/12* (2006.01)
*C07D 413/12* (2006.01) *C07D 403/12* (2006.01)
*C07D 405/12* (2006.01) *C07D 417/12* (2006.01)
*A61K 31/33* (2006.01) *A61P 29/00* (2006.01)

(86) Numéro de dépôt international:
**PCT/FR2006/000008**

(87) Numéro de publication internationale:
**WO 2006/072736 (13.07.2006 Gazette 2006/28)**

(54) **DERIVES DE N-(HETEROARYL)-1H-INDOLE-2-CARBOXAMIDES ET LEUR UTILISATION COMME LIGANDS DU RECEPTEUR VANILLOIDE TRPV1**

N-(HETEROARYL)-1H-INDOL-2-CARBONSÄUREAMIDDERIVATE UND DEREN VERWENDUNG ALS VANILLOID-TRPV1-REZEPTORLIGANDEN

N-(HETEROARYL)-1H-INDOLE-2-CARBOXAMIDE DERIVATIVES AND THEIR USE AS VANILLOID TRPV1 RECEPTOR LIGANDS

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **07.01.2005 FR 0550068**

(43) Date de publication de la demande:
**10.10.2007 Bulletin 2007/41**

(73) Titulaire: **Sanofi-Aventis**
**75013 Paris (FR)**

(72) Inventeurs:
- **DUBOIS, Laurent**
  **F-92350 Le Plessis-Robinson (FR)**
- **EVANNO, Yannick**
  **F-78830 Bullion (FR)**
- **MALANDA, André**
  **F-91140 Villejust (FR)**

(74) Mandataire: **Morel-Pécheux, Muriel et al**
**sanofi-aventis**
**Département Brevets**
**174 avenue de France**
**75013 Paris (FR)**

(56) Documents cités:
**WO-A-03/068749**
**WO-A-2004/072069**
**WO-A-2004/096784**
**WO-A-2004/108133**
**WO-A-2006/024776**
**US-A1- 2005 165 049**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 841 737 B1

Printed by Jouve, 75001 PARIS (FR)

## Description

L'invention a pour objet des composés dérivés de *N*-(hétéroaryl)-1*H*-indole-2-carboxamides, qui présentent une activité antagoniste ou agoniste *in vitro* et *in vivo* pour les récepteurs de type TRPV1 (ou VR1).

Un premier objet de l'invention concerne les composés répondant à la formule générale . (I) ci-après.
Un autre objet de l'invention concerne des procédés de préparation des composés de formule générale (I).
Un autre objet de l'invention concerne l'utilisation des composés de formule générale (I) notamment dans des médicaments ou dans des compositions pharmaceutiques.

Les composés de invention répondent à la formule générale (I) :

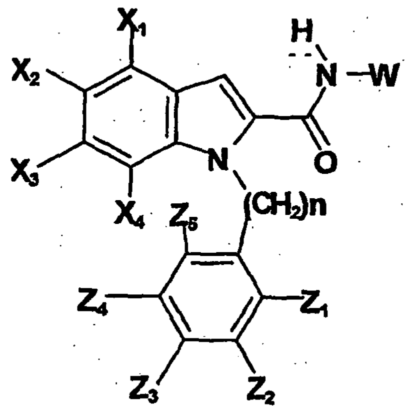

(I)

dans laquelle

n est égal à 0, 1, 2 ou 3;
$X_1$, $X_2$, $X_3$, $X_4$, $Z_1$, $Z_2$, $Z_3$, $Z_4$ et $Z_5$ représentent, indépendamment l'un de l'autre, un atome d'hydrogène ou d'halogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxy, $C_1$-$C_6$-fluoroalcoxy, cyano, C(O)$NR_1R_2$, nitro, $NR_1R_2$, $C_1$-$C_6$-thloalkyle, -S(O)-$C_1$-$C_6$-alkyle, -$S(O)_2$-$C_1$-$C_6$-alkyle, $SO_2NR_1R_2$, $NR_3COR_4$, $NR_3SO_2R_5$ ou aryle, l'aryle étant éventuellement substitué par un ou plusieurs substitutants choisi parmi un halogène, un groupe $C_1$-$C_6$-alkyle, $C_3$-$C_7$-cycloalkyle, $C_3$-$C_7$-cycloalkyte-$C_1$-$C_3$-alkylène, $C_1$-$C_6$-fluoroalkyle, $C_1$-$C_6$-alcoxy, $C_1$-$C_6$-fluoroalcoxy, nitro ou cyano ;
$R_1$ et $R_2$, représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_3$-alkylène ou aryle ; ou $R_1$ et $R_2$ formant ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe $C_1$-$C_6$-alkyle, $C_3$-$C_7$-cycloalkyle, $C_3$-$C_7$-cycloalkyle-$C_1$-$C_3$-alkylène ou aryle ;
$R_3$ et $R_4$ représentent, indépendamment l'un de l'autre, un atome d'hydrogène ou un groupe $C_1$-$C_6$-alkyle ou aryle ;
$R_5$ représente un groupe $C_1$-$C_6$-alkyle ou aryle ;
W représente un groupe bicyclique fusionné de formule :

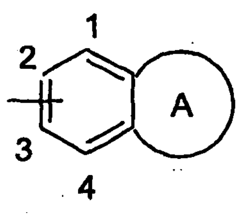

lié à l'atome d'azote par les positions 1, 2, 3 ou 4 ;
A représente un hétérocycle de 5 à 7 chaînons comprenant de un à trois hétéroatomes choisi parmi O, S ou N ;
le ou les atomes de carbones de A étant éventuellement substitués par un ou plusieurs groupes choisis parmi un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle, aryle-$C_1$-$C_6$-alkylène, oxo ou thio;
le ou les atomes d'azote de A étant éventuellement substitués par $R_6$ lorsque l'azote est adjacent à un atome de

carbone substitué par un groupe oxo, ou par $R_7$ dans les autres cas ;
$R_6$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène ou aryle ;
$R_7$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène, $C_1$-$C_6$-alkyle-C(O)-, $C_3$-$C_7$-cycloalkyle-$C_1$-$C_3$-alkylène-(CO)-, $C_1$-$C_6$-fluoroalkyle-C(O)-, $C_3$-$C_7$-cycloalkyle-C(O)-, aryle-C(O)-, aryle-$C_1$-$C_6$-alkylène-C(O)-, $C_1$-$C_6$-alkye-$S(O)_2$-, $C_1$-$C_6$-fluoroalkyle-$S(O)_2$-, $C_3$-$C_7$-cycloalkyle-$S(O)_2$-, $C_3$-$C_7$-cycloalkyle-$C_1$-$C_3$-alkylène-$S(O)_2$-, aryle-$S(O)_2$- ou aryle-$C_1$-$C_6$-alkylène-$S(O)_2$- ou aryle ; et
W est différent de l'indolyle.

Dans les composés de formule générale (I) :

- le ou les atomes de soufre de l'hétérocycle A peuvent être sous forme oxydée (S(O) ou $S(O)_2$);
- le ou les atomes d'azote de l'hétérocycle A peuvent être sous forme oxydée (N-oxyde).

Parmi les composés de formule générale (I) objets de l'invention, un premier sous-groupe de composés est constitué par les composés pour lesquels n est égal à 0 ou 1.

Parmi les composés de formule générale (I) objets de l'invention, un second sous-groupe de composés est constitué par les composés pour lesquels $X_1$, $X_2$; $X_3$, $X_4$, $Z_1$, $Z_2$, $Z_3$, $Z_4$ et $Z_5$ représentent, indépendamment l'un de l'autre, un atome d'hydrogène ou d'halogène, plus particulièrement un fluor, ou un groupe $C_1$-$C_6$-alkyle; plus particulièrement un méthyle, ou un groupe $C_1$-$C_6$-fluoroalkyle, plus particulièrement un $CF_3$, ou un groupe $C_1$-$C_6$-alcoxy , plus particulièrement un méthoxy.

Parmi les composés de formule générale (I) objets de l'invention, un troisième sous-groupe de composés est constitué par les composés pour lesquels W est choisi parmi les groupes indolinyle, isoindolinyle, benzofuranyle, dihydrobenzofuranyle, benzothiophényle, dihydrobenzothiophényle, benzoxazolyle, dihydrobenzoxazolinyle, isobenzofuranyle, dihydroisobenzofuranyle, benzimidazolyle, dihydrobenzimidazolyle, indazolyle, benzothiazolyle, isobenzothiazolyle, dihydroisobenzothiazolyle, benzotriazolyle, quinoléinyle, dihydroquinoléinyle, tétrahydroquinotéinyle; isoquinoléinyle, dihydroisoquinoléinyle, tétrahydroisoquinoléinyle, benzoxazinyle, dihydrobenzoxazinyle, benzothiazinyle, dihydrobenzothiazinyle, cinnolinyle, quinazolinyle, dihydroquinazolinyle, tétrahydroquinazolinyle, quinoxalinyle, dihydroquinoxalinyle, tétrahydroquinoxalinyle, phtalazinyle, dihydrophtalazinyle, tétrahydrophtalazinyle, tétrahydrobenz[*b*]azépinyle, tétrahydrobenz[*c*]azépinyle, tétrahydrobenz[*d*]azépinyle, tétrahydrobenzo[*b*][1,4]diazépinyle, tétrahydrobenzo[*e*][1,4]diazépinyle, tétrahydrobenzo[*b*][1,4]oxazépinyle ou tétrahydrobenzo[*b*][1,4]thiazépinyle ; le ou les atomes de carbone et/ou d'azote dudit groupe W étant éventuellement substitués comme défini dans la formule générale (I).

Parmi les composés du troisième sous-groupe, un quatrième sous-groupe de composés est constitué par les composés pour lesquels W est choisi parmi les groupes isoquinoléinyle, dihydroquinoléinyle, tétrahydroquinoléinyle, benzoxazinyle, dihydrobenzoxazinyle, benzofuranyle, indolinyle, benzoxazolyle, indazolyle, benzimidazolyle, benzothiazolyle, benzotriazolyle, quinoléinyle, quinoxalinyle ; le ou les atomes de carbone dudit groupe W étant éventuellement substitués par un ou plusieurs groupes choisis parmi un groupe oxo, $C_1$-$C_6$-alkyle, plus particulièrement méthyle ou éthyle, ou aryle, plus particulièrement phényle, tels que définis dans la formule générale (I) en relation avec A ; et/ou le ou les atomes d'azote dudit groupe W étant éventuellement substitués par $R_6$ lorsque l'azote est adjacent à un atome de carbone substitué par un groupe oxo, ou par $R_7$ dans les autre cas, $R_6$ et $R_7$ étant tels que définis dans la formules générale (I) en relation avec A,
avec $R_6$ représentant un atome d'hydrogène ou un groupe $C_1$-$C_6$-alkyle, plus particulièrement un méthyle,
avec $R_7$ représentant un atome d'hydrogène ou un groupe $C_1$-$C_6$-alkyle, plus particulièrement un méthyle, ou un $C_1$-$C_6$-alkyle-$S(O)_2$-, plus particulièrement un méthylsulfonyle.

Un cinquième sous-groupe de composés est constitué par les composés de formule générale (I) :

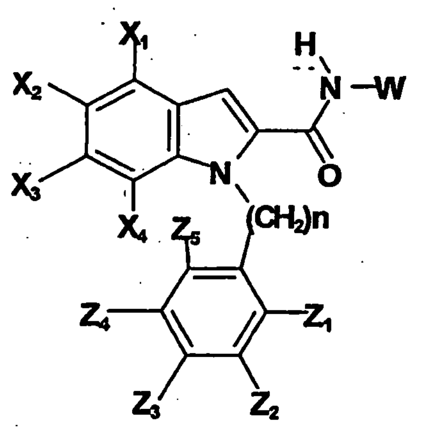

(I)

dans laquelle

n est égal à 0, 1, 2 ou 3;

$X_1$, $X_2$, $X_3$, $X_4$, $Z_1$, $Z_2$, $Z_3$, $Z_4$ et $Z_5$ représentent, indépendamment l'un de l'autre, un atome d'hydrogène ou d'halogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxy, $C_1$-$C_6$-fluoroalcoxy, cyano, $C(O)NR_1R_2$, nitro, $NR_1R_2$, $C_1$-$C_6$-thioalkyle, -S(O)-$C_1$-$C_6$-alkyle, -$S(O)_2$-$C_1$-$C_6$-alkyle, $SO_2NR_1R_2$, $NR_3COR_4$, $NR_3SO_2R_5$ ou aryle, l'aryle étant éventuellement substitué par un ou plusieurs substitutants choisi parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxy, $C_1$-$C_6$-fluoroalcoxy, nitro ou cyano ;

$R_1$ et $R_2$, représentent, indépendamment l'un de l'autre, un atome d'hydrogène ou un groupe $C_1$-$C_6$-alkyle, $C_3$-$C_7$-cydoalkyle, $C_3$-$C_7$-cycloalkyle-$C_1$-$C_3$-alkylène ou aryle ; ou $R_1$ et $R_2$ formant ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe $C_1$-$C_6$-alkyle, $C_3$-$C_7$-cycloalkyle, $C_3$-$C_7$-cycloalkyle-$C_1$-$C_3$-alkylène ou aryle ;

$R_3$ et $R_4$ représentent, indépendamment l'un de l'autre, un atome d'hydrogène ou un groupe $C_1$-$C_6$-alkyle ou aryle ;

$R_5$ représente un groupe $C_1$-$C_6$-alkyle ou aryle ;

W représente un groupe bicyclique fusionné de formule :

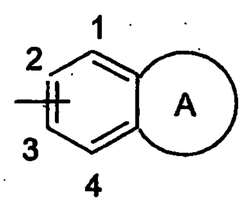

lié à l'atome d'azote par les positions 1, 2, 3 ou 4 ;

A représente un hétérocycle de 5 à 7 chaînons comprenant de un à trois hétéroatomes choisi parmi O, S ou N ;

le ou les atomes de carbones de A étant éventuellement substitués par un ou plusieurs groupes choisis parmi un atome d'hydrogène où un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle, aryle-$C_1$-$C_6$-alkylène, oxo ou thio;

le ou les atomes d'azote de A étant éventuellement substitués par $R_6$ lorsque l'azote est adjacent à un atome de carbone substitué par un groupe oxo, ou par $R_7$ dans les autres cas ;

$R_6$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène ou aryle ;

$R_7$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène, $C_1$-$C_6$-alkyle-C(O)-, $C_3$-$C_7$-cycloalkyle-$C_1$-$C_3$-alkylène-(CO)-, $C_1$-$C_6$-fluoroalkyle-C(O)-, $C_3$-$C_7$-cycloalkyle-C(O)-, aryle-C(O)-, aryle-$C_1$-$C_6$-alkylène-C(O)-, $C_1$-$C_6$-alkyle-$S(O)_2$-, $C_1$-$C_6$-fluoroalkyle-$S(O)_2$-, $C_3$-$C_7$-cycloalkyle-$S(O)_2$-, $C_3$-$C_7$-cycloalkyle-$C_1$-$C_3$-alkylène-$S(O)_2$-, aryle-$S(O)_2$- ou aryle-$C_1$-$C_8$-alkylène-$S(O)_2$- ou aryle ; et

W est différent de l'indolyle ;

à la condition que lorsque $Z_1$, $Z_2$, $Z_3$, $Z_4$ et $Z_5$ représentent simultanément des atomes d'hydrogène, alors n = 2 ou 3.

Parmi les composés de formule générale (I) objets de l'invention, un sixième sous-groupe de composés est constitué par les composés pour lesquels W est différent des groupes quinoléinyle, dihydroquinoléinyle, tétrahydroquinoléinyle, isoquinoléinyle, dihydroisoquinoléinyle ou tétrahydroisoquinoléinyle.

Parmi les composés de formule générale (I) objets de l'invention, un septième sous-groupe de composés est constitué par l'ensemble des composés de la formule générale (I) :

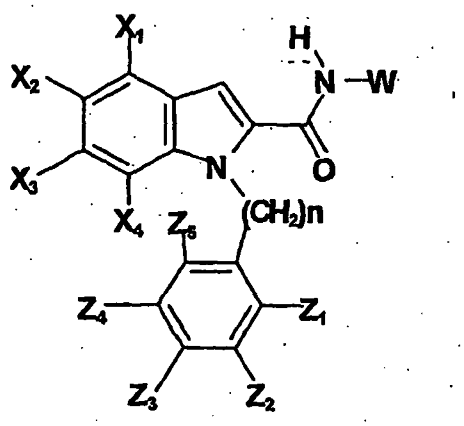

(I)

dans laquelle

n est égal à 0, 1, 2 ou 3;
$N_1$, $X_3$, $X_4$, $Z_1$, $Z_3$, $Z_4$ et $Z_5$ représentent des atomes d'hydrogène, $X_2$ représente un atome d'hydrogène, un atome de fluor ou un groupé $CF_3$ et $Z_2$ représente un atome d'hydrogène ou un atome de fluor ;
$R_1$ et $R_2$, représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_3$-alkyléne ou aryle ; ou $R_1$ et $R_2$ formant ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe $C_1$-$C_6$-alkyle, $C_3$-$C_7$-cycloalkyle, $C_3$-$C_7$-cycloalkyle-$C_1$-$C_3$-alkylène ou aryle ;
$R_3$ et $R_4$ représentent, indépendamment l'un de l'autre, un atome d'hydrogène ou un groupe $C_1$-$C_6$-alkyle ou aryle ;
$R_5$ représente un groupe $C_1$-$C_6$-alkyle ou aryle ;
W représente un groupe bicyclique fusionné de formule :

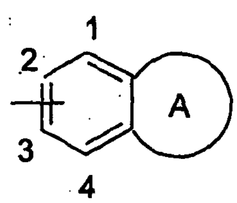

lié à l'atome d'azote par les positions 1, 2, 3 ou 4 ;
A représente un hétérocycle de 5 à 7 chaînons comprenant de un à trois hétéroatomes choisi parmi O, S ou N;
le ou les atomes de carbones de A étant éventuellement substitués par un ou plusieurs groupes choisis parmi un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryl, aryle-$C_1$-$C_6$-alkylène, oxo ou thio;
le ou les atomes d'azote de A étant éventuellement substitués par $R_6$ lorsque l'azote est adjacent à un atome de carbone substitué par un groupe oxo, ou par $R_7$ dans les autres cas ;
$R_6$ représente un atome d'hydrogène ou un groupe $C_1$-$C_6$-alkyle, $C_3$-$C_7$-cycloalkyle, $C_3$-$C_7$-cycloalkyle-$C_1$-$C_3$alkylène, $C_1$-$C_6$-fluoroalkyle, aryle-$C_1$-$C_6$-alkylène ou aryle ;
$R_7$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène, $C_1$-$C_6$-alkyle-C(O)-, $C_3$-$C_7$-cycloalkyle-$C_1$-$C_3$-alkylène-(CO)-, $C_1$-$C_6$-fluoroalkyle-C(O)-, $C_3$-$C_7$-cycloalkyle-C(O)-, aryle-C(O)-, aryle-$C_1$-$C_6$-alkylène-C(O)-, $C_1$-$C_6$-alkyle-$S(O)_2$-, $C_1$-$C_6$-fluoroalkyle-$S(O)_2$-, $C_3$-$C_7$-cycloalkyle-$S(O)_2$-, $C_3$-$C_7$-cycloalkyle-$C_1$-$C_3$-alkylène-$S(O)_2$-, aryle-$S(O)_2$- ou aryle-$C_1$-$C_6$-alkylène-$S(O)_2$- ou aryle ; et
W est différent de l'indolyle.

Parmi les composés de formule générale (I) objets de l'invention, un huitième sous-groupe de composés est constitué par les composés pour lesquels W est tel que défini dans le sixième sous-groupe ci-dessus et $X_1$, $X_2$, $X_3$, $X_4$, $Z_1$, $Z_2$, $Z_3$, $Z_4$ et $Z_5$ sont tels que définis dans le septième sous-groupes ci-dessus.

Parmi les composés de formule générale (I) objets de l'invention, un neuvième sous-groupe de composés est constitué par l'ensemble des composés de la formule générale (I) :

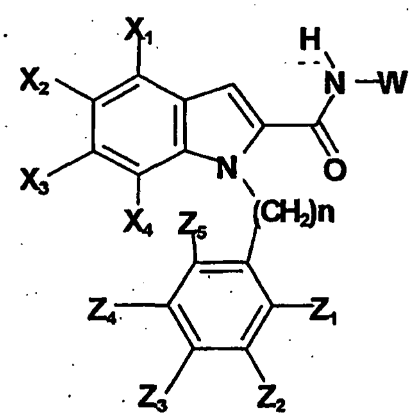

(I)

dans laquelle

n est égal à 0, 1, 2 ou 3 ;

$X_1$, $X_2$, $X_3$, $X_4$, $Z_1$, $Z_2$, $Z_3$, $Z_4$ et $Z_5$ représentent, indépendamment l'un de l'autre, un atome d'hydrogène ou d'halogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxy, $C_1$-$C_6$-fluoroalcoxy, cyano, $C(O)NR_1R_2$, nitro, $NR_1R_2$; $C_1$-$C_6$-thioalkyle, -S(O)-$C_1$-$C_6$-alkyle, -$S(O)_2$-$C_1$-$C_6$-alkyle, $SO_2NR_1R_2$, $NR_3COR_4$, $NR_3SO_2R_5$ ou aryle, l'aryle étant éventuellement substitué par un ou plusieurs substitutants choisi parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxy, $C_1$-$C_6$-fluoroalcoxy, nitro ou cyano ;

$R_1$ et $R_2$, représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_3$-alkylène ou aryle ou $R_1$ et $R_2$ formant ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe $C_1$-$C_6$-alkyle, $C_3$-$C_7$-cycloalkyle, $C_3$-$C_7$-cycloalkyle-$C_1$-$C_3$-alkylène ou aryle ;

$R_3$ et $R_4$ représentent, indépendamment l'un de l'autre, un atome d'hydrogène ou un groupe $C_1$-$C_6$-alkyle ou aryle ;

$R_5$ représente un groupe $C_1$-$C_6$-alkyleou aryle ;

W représente un groupe bicyclique fusionné de formule :

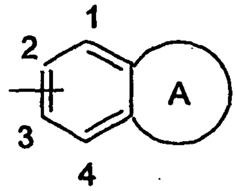

lié à l'atome d'azote par les positions 1, 2, 3 ou 4 ;

A représente un hétérocycle de 5 à 7 chaînons comprenant de un à trois hétéroatomes choisi parmi O, S ou N ;

le ou les atomes de carbones de A étant éventuellement substitués par un ou plusieurs groupes choisis parmi un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle, aryle-$C_1$-$C_6$-alkylène, oxo ou thio;

le ou les atomes d'azote de A étant éventuellement substitués par $R_6$ lorsque l'azote est adjacent à un atome de carbone substitué par un groupe oxo, ou par $R_7$ dans les autres cas ;

$R_6$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène ou aryle ;

$R_7$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène, $C_1$-$C_6$-alkyle-C(O)-, $C_3$-$C_7$-cycloalkyle-$C_1$-$C_3$-alkylène-(CO)-,

$C_1$-$C_6$-fluoroalkyle-C(O)-, $C_3$-$C_7$-cycloalkyle-C(O)-, aryle-C(O)-, aryle-$C_1$-$C_6$-alkylène-C(O)-, $C_1$-$C_6$-alkyle-$S(O)_2$-, $C_1$-$C_6$-fluoroalkyle-$S(O)_2$-, $C_3$-$C_7$-cycloalkyle-$S(O)_2$, $C_3$-$C_7$-cycloalkyle-$C_1$-$C_3$-alkylène-$S(O)_2$-, aryle-$S(O)_2$- ou aryle-$C_1$-$C_6$-alkylène-$S(O)_2$- ou aryle ; et

W est différent de l'indolyle,

les composés suivants étant exclus : N-(quinoléin-7-yl)-1-benzyl-6-bromo-1H-indole-2-carboxamide, N-(quinoléin-7-yl)-1-benzyl-5-bromo-1H-indole-2-carboxamide et N-(quinoléin-7-yl)-6-bromo-1-(4-(trifluoromethyl)benzyl)-1H-indole-2-carboxamide. Ces trois composés sont décrits dans le document US 2005/0165049.

Parmi les composés de formule générale (I) objets de l'invention, un dixième sous-groupe de composés est constitué par l'ensemble des composés de la formule générale (I):

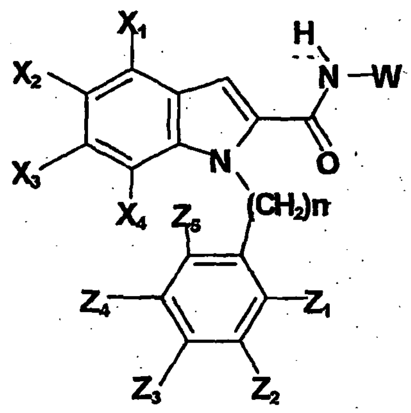

(I)

dans laquelle

n est égal à 0, 1, 2 ou 3;

$X_1$, $X_2$, $X_3$, $X_4$, $Z_1$, $Z_2$, $Z_3$, $Z_4$ et $Z_5$ représentent, indépendamment l'un de l'autre, un atome d'hydrogène ou d'halogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxy, $C_1$-$C_6$-fluoroalcoxy, cyano, $C(O)NR_1R_2$, nitro, $NR_1R_2$, $C_1$-$C_6$-thioalkyle, -S(O)-$C_1$-$C_6$-alkyle, -$S(O)_2$-$C_1$-$C_6$-alkyle, $SO_2NR_1R_2$, $NR_3COR_4$, $NR_3SO_2R_5$ ou aryle, l'aryle étant éventuellement substitué par un ou plusieurs substitutants choisi parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxy, $C_1$-$C_6$-fluoroalcoxy, nitro ou cyano ;

$R_1$ et $R_2$, représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_3$-alkylène ou aryle ; ou $R_1$ et $R_2$ formant ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe $C_1$-$C_6$-alkyle, $C_3$-$C_7$-cycloalkyle, $C_3$-$C_7$-cycloalkyle-$C_1$-$C_3$-alkylène ou aryle ;

$R_3$ et $R_4$ représentent, indépendamment l'un de l'autre, un atome d'hydrogène ou un groupe $C_1$-$C_6$-alkyle ou aryle;

$R_5$ représente un groupe -$C_1$-$C_8$-alkyle ou aryle ;

W représente un groupe bicyclique fusionné de formule :

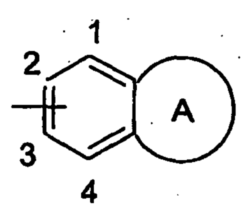

lié à l'atome d'azote par les positions 1, 2, 3 ou 4 ;

A représente un hétérocycle de 5 à 7 chaînons comprenant de un à trois hétéroatomes choisi parmi O, S ou N ;

le ou les atomes de carbones de A étant éventuellement substitués par un ou plusieurs groupes choisis parmi un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle, aryle-$C_1$-$C_8$-alkylène; oxo ou thio;

le ou les atomes d'azote de A étant éventuellement substitués par $R_6$ lorsque l'azote est adjacent à un atome de

carbone substitué par un groupe oxo, ou par $R_7$ dans les autres cas ;

représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène ou aryle ;

$R_7$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène, $C_1$-$C_6$-alkyle-C(O)-, $C_3$-$C_7$-cycloalkyle-$C_1$-$C_3$-alkylène-(CO)-, $C_1$-$C_6$-fluoroalkyle-C(O)-, $C_3$-$C_7$-cycloalkyle-C(O)-, aryle-C(O)-, aryle-$C_1$-$C_6$-alkylène-C(O)-, $C_1$-$C_6$-alkyle-$S(O)_2$-, $C_1$-$C_6$-fluoroalkyle-$S(O)_2$-, $C_3$-$C_7$-cycloalkyle-$S(O)_2$-, $C_3$-$C_7$-cycloalkyle-$C_1$-$C_3$-alkylène-$S(O)_2$-, aryle-$S(O)_2$- ou aryle-$C_1$-$C_6$-alkylène-S(O)2- ou aryle ; et

W est différent de l'indolyle ;

à la condition que lorsque W est un groupe benzimidazolyle, benzothiazolyle ou benzoxazolyle, alors $Z_1$, $Z_2$, $Z_3$, $Z_4$ et $Z_5$ représentent un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-fluoroalcoxy, cyano, C(O)$NR_1R_2$, -S(O)-$C_1$-$C_6$-alkyle, $SO_2NR_1R_2$, $NR_3COR_4$, $NR_3SO_2R_5$ ou aryle.

Parmi les composés de formule générale (I) objets de l'invention, un onzième sous-groupe de composés est constitué par l'ensemble des composés de la formule générale (I):

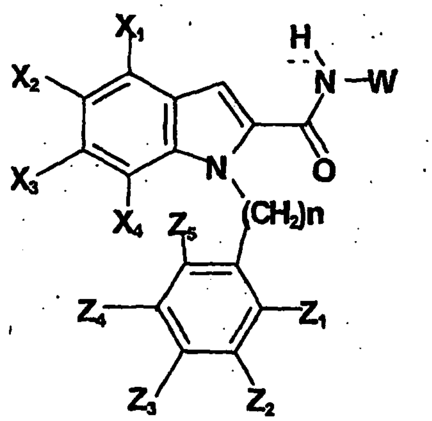

(I)

dans laquelle

n est égal à 0, 1, 2 ou 3;

$X_1$, $X_2$, $X_3$, $X_4$, $Z_1$, $Z_2$, $Z_3$, $Z_4$ et $Z_5$ représentent, indépendamment l'un de l'autre, un atome d'hydrogène ou d'halogène ou un groupe $C_1$-$C_6$-alkyle, $C_3$-$C_7$-cycloalkyle;. $C_3$-$C_7$-cycloalkyle-$C_1$-$C_3$-alkylène, $C_1$-$C_6$-fluoroalkyle, $C_1$-$C_6$-alcoxy, $C_1$-$C_6$-fluoroalcoxy, cyano, C(O)$NR_1R_2$, nitro, $NR_1R_2$, $C_1$-$C_6$-thioalkyle, -S(O)-$C_1$-$C_6$-alkyle, -$S(O)_2$-$C_1$-$C_6$-alkyle, $SO_2NR_1R_2$, $NR_3COR_4$, $NR_3SO_2R_5$ ou aryle, l'aryle étant éventuellement substitué par un ou plusieurs substitutants choisi parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxy, $C_1$-$C_6$-fluoroalcoxy, nitro ou cyano ;

$R_1$ et $R_2$, représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_3$-alkylène ou aryle ; ou $R_1$ et $R_2$ formant ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe $C_1$-$C_6$-alkyle, $C_3$-$C_7$-cycloalkyle, $C_3$-$C_7$-cycloalkyle-$C_1$-$C_3$-alkyléne ou aryle ;

$R_3$ et $R_4$ représentent, indépendamment l'un de l'autre, un atome d'hydrogène ou un groupe $C_1$-$C_6$-alkyle ou aryle ;

$R_5$ représente un groupe $C_1$-$C_6$-alkyle ou aryle ;

W représente un groupe bicyclique fusionné de formule :

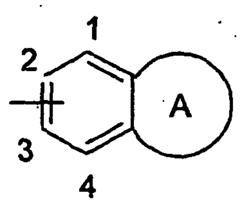

lié à l'atome d'azote par les positions 1, 2, 3 ou 4 ;
A représente un hétérocycle de 5 à 7 chaînons comprenant de un à trois hétéroatomes choisi parmi O, S ou N ;
le ou les atomes de carbones de A étant éventuellement substitués par un ou plusieurs groupes choisis parmi un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle, aryle-$C_1$-$C_6$-alkylène, oxo ou thio;
le ou les atomes d'azote de A étant éventuellement substitués pas lorsque l'azote est adjacent à un atome de carbone substitué par un groupe oxo, ou par $R_7$ dans les autres cas ;
$R_6$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène ou aryle ;
$R_7$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène, $C_1$-$C_6$-alkyle-C(O)-, $C_3$-$C_7$-cycloalkyle-$C_1$-$C_3$-alkylène-(CO)-, $C_1$-$C_6$-fluoroalkyle-C(O)-, $C_3$-$C_7$-cycloalkyle-C(O)-, aryle-C(O)-, aryle-$C_1$-$C_6$-alkylène-C(O)-, $C_1$-$C_6$-alkyle-$S(O)_2$-, $C_1$-$C_6$-fluoroalkyle-$S(O)_2$-, $C_3$-$C_7$-cycloalkyle-$S(O)_2$-, $C_3$-$C_7$-cycloalkyle-$C_1$-$C_3$-alkylène-$S(O)_2$-, aryle-$S(O)_2$- ou aryle-$C_1$-$C_6$-alkylène-$S(O)_2$- ou aryle ; et
W est différent de l'indolyle
à la condition que lorsque A est un hétérocycle à 5 chaînons, alors il est insaturé.

Parmi les composés de formule générale (I) objets de l'invention, un douzième sous-groupe de composés est constitué par l'ensemble des composés de la formule générale (I) :

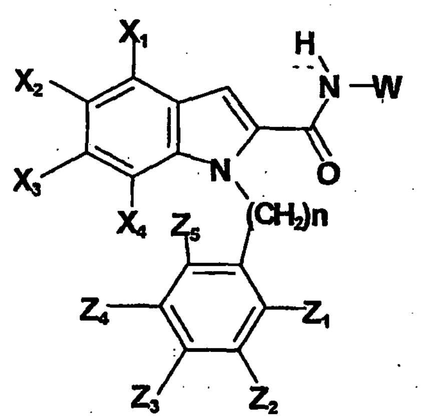

(I)

dans laquelle

n est égal à 0, 1, 2 ou 3;
$X_1$, $X_2$, $X_3$, $X_4$, $Z_1$, $Z_2$, $Z_3$, $Z_4$ et $Z_5$ représentent, indépendamment l'un de l'autre, un atome d'hydrogène ou d'halogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxy, $C_1$-$C_6$-fluoroalcoxy, cyano, C(O)$NR_1R_2$, nitro, $NR_1R_2$, $C_1$-$C_6$-thioalkyle, -S(O)-$C_1$-$C_6$-alkyle, -$S(O)_2$-$C_1$-$C_6$-alkyle, $SO_2NR_1R_2$, $NR_3COR_4$, $NR_3SO_2R_5$ ou aryle, l'aryle étant éventuellement substitué par un ou plusieurs substitutants choisi parmi un halogène, un groupe $C_1$-$C_8$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_8$-alcoxy, $C_1$-$C_6$-fluoroalcoxy, nitro ou cyano ;
$R_1$ et $R_2$, représentent, indépendamment l'un de l'autre; un atome d'hydrogène ou un groupe $C_1$-$C_6$-alkyle, $C_3$-$C_7$-cycloalkyle, $C_3$-$C_7$-cycloalkyle-$C_1$-$C_3$-alkylène ou aryle ; ou $R_1$ et $R_2$ formant ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe $C_1$-$C_8$-alkyle, $C_3$-$C_7$-cycloalkyle, $C_3$-$C_7$-cycloalkyle-$C_1$-$C_3$-alkylène ou aryle ;
$R_3$ et $R_4$ représentent, indépendamment l'un de l'autre, un atome d'hydrogène ou un groupe $C_1$-$C_6$-alkyle ou aryle ;
$R_5$ représente un groupe $C_1$-$C_6$-alkyle ou aryle ;
W représente un groupe bicyclique fusionné de formule:

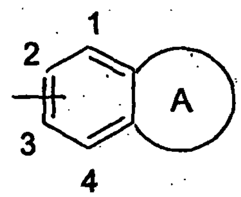

lié à l'atome d'azote par les positions 1, 2, 3 ou 4 :

A représente un hétérocycle de 5 à 7 chaînons comprenant de un à trois hétéroatomes choisi parmi O, S ou N ;

le ou les atomes de carbones de A étant éventuellement substitués par un ou plusieurs groupes choisis parmi un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle, aryle-$C_1$-$C_6$-alkylène, oxo ou thio;

le ou les atomes d'azote de A étant éventuellement substitués par $R_6$ lorsque l'azote est adjacent à un atome de carbone substitué par un groupe oxo, ou par $R_7$ dans les autres cas;

$R_6$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène ou aryle;

$R_7$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène, $C_1$-$C_6$-alkyle-C(O)-, $C_3$-$C_7$-cycloalkyle-$C_1$-$C_3$-alkylène-(CO)-, $C_1$-$C_6$-fluoroalkyle-C(O)-, $C_3$-$C_7$-cycloalkyle-C(O)-, aryle-C(O)-, aryle-$C_1$-$C_6$-alkylène-C(O)-, $C_1$-$C_6$-alkyle-$S(O)_2$-, $C_1$-$C_6$-fluoroalkyle-$S(O)_2$-, $C_3$-$C_7$-cycloalkyle-$S(O)_2$-, $C_3$-$C_7$-cycloalkyle-$C_1$-$C_3$-alkylène-$S(O)_2$-, aryle-$S(O)_2$- ou aryle-$C_1$-$C_6$-alkylène-$S(O)_2$- ou aryle ; et

W est différent de l'indolyle

à la condition que Au soit différent d'un hétérocycle à 5 chaînons insaturé.

Les composés pour lesquels à la fois n, $X_1$, $X_2$, $X_3$, $X_4$, $Z_1$, $Z_2$, $Z_3$. $Z_4$, $Z_5$ et W sont tels que définis dans les sous-groupes de composés ci-dessus, forment un treizième sous-groupe.

Parmi les composés de formule générale (I) objets de l'invention, un quatorzième sous-groupe de composés est constitué par les composés suivants :

*N*-(isoquinoléin-5-yl)-5-fluoro-1-[((3-trifluorométhyl)phényl)méthyl]-1*H*-indole-2-carboxamide,
*N*-(1-méthyl-1,2,3,4-tétrahydroquinoléin-7-yl)-1-[3-(trifluorométhyl)phényl]-1*H*-indole-2-carboxamide,
*N*-(1-méthyl-1,2,3,4-tétrahydroquinoléin-7-yl)-1-((3-5-diméthyl)phényl)-1*H*-indole-2-carboxamide,
*N*-(1,2,3,4-tétrahydroquinoléin-7-yl)-1-[3-(trifluorométhyl)phényl]-1*H*-indole-2-carboxamide,
*N*-(4-méthyl-3-oxo-2*H*-benzoxazin-7-yl)-1-[3-(trifluorométhyl)phényl]-1*H*-indole-2-carboxamide,
*N*-(4-méthyl-3-oxo-2*H*-benzoxazin-6-yl)-1-[3-(trifluorométhyl)phényl]-1*H*-indole-2-carboxamide,
*N*-(2-oxo-3,4-dihydroquinoléin-7-yl)-1-[3-(trifluorométhyl)phényl]-1*H*-indole-2-carboxamide,
*N*-(benzofuran-5-yl)-1-[3-(trifluorométhyl)phényl]-1*H*-indole-2-carboxamide,
*N*-(1-méthyl-indolin-5-yle)-1-[3-(trifluorométhyl)phényl]-1*H*-indole-2-carboxamide,
*N*-(2,3-dihydrobenzoxazin-6-yl)-1-[3-(trifluorométhyl)phényl]-1*H*-indole-2-carboxamide,
*N*-(3-oxo-2H-benzoxazin-7-yl)-1-[3-(trifluorométhyl)phényl]-1*H*-indole-2-carboxamide,
*N*-(1-méthyl-indolin-5-yl)-5-fluoro-1-[(3-fluorophényl)méthyl]-1*H*-indole-2-carboxamide,
*N*-(1-méthyl-1,2,3,4-tétrahydroquinoléin-7-yl)-5-fluoro-1-[(3-fluorophényl)méthyl]-1*H*-indole-2-carboxamide,
*N*-(1,2,3,4-tétrahydroquinoléin-7-yl)-5-fluoro-1-[(3-fluorophényl)méthyl]-1*H*-indole-2-carboxamide,
*N*-(isoquinoléin-5-yl)-5-fluoro-1-[(3-fluorophényl)méthyl]-1*H*-indole-2-carboxamide,
*N*-(1,2,3,4-tétrahydroquinoléin-8-yl)-1-[3-(trifluorométhyl)phényl]-1*H*-indole-2-carboxamide,
*N*-(benzoxazol-5-yl)-1-[3-(trifluorométhyl)phényl]-1*H*-indole-2-carboxamide,
*N*-(2-méthyl-benzoxazol-5-yl)-1-[3-(trifluorométhyl)phényl]-1*H*-indole-2-carboxamide,
*N*-(1-méthyl-1*H*-indazol-5-yl)-5-trifluorométhyl-1-[(3-fluorophényl)méthyl]-1*H*-indole-2-carboxamide,
*N*-(2-oxo-3,4-dihydroquinoléin-7-yl)-5-fluoro-1-[(3-fluorophényl)méthyl]-1*H*-indole-2-carboxamide,
*N*-(benzofuran-5-yl)-5-fluoro-1-[(3-fluorophényl)méthyl]-1*H*-indole-2-carboxamide,
*N*-(2,3-dihydrobenzoxazin-6-yl)-5-fluoro-1-[(3-fluorophényl)méthyl]-1*H*-indole-2-carboxamide,
*N*-(3-oxo-2*H*-benzoxazin-6-yl)-5-fluoro-1-[(3-fluorophényl)méthyl]-1*H*-indole-2-carboxamide,
*N*-(1,2,3,4-tétrahydroquinoléin-7-yl)-5-trifluorométhyl-1-[(3-fluorophényl)méthyl]-1*H*-indole-2-carboxamide,
*N*-(2-oxo-indolin-5-yl)-5-trifluorométhyl-1-[(3-fluorophényl)méthyl]-1*H*-indole-2-carboxamide,
*N*-(1-méthyl-benzimidazol-5-yl)-5-trifluorométhyl-1-[(3-fluorophényl)méthyl]-1*H*-indole-2-carboxamide,
*N*-(1-méthyl-1,2,3,4-tétrahydroquinoléin-7-yl)-5-trifluorométhyl-1-[(3-fluorophényl)méthyl]-1*H*-indole-2-carboxamide,

*N*-(benzothiazol-6-yl)-5-trifluorométhyl-1-[(3-fluorophényl)méthyl]-1*H*-indole-2-carboxamide,
*N*-(2-méthyl-benzoxazol-5-yl)-5-trifluorométhyl-1-[(3-fluorophényl)méthyl]-1*H*-indole-2-carboxamide,
*N*-(2-méthyl-benzothiazol-5-yl)-5-trifluorométhyl-1-[(3-fluorophényl)méthyl]-1*H*-indole-2-carboxamide,
*N*-(1-méthylsulfonyl-indolin-5-yl)-5-trifluorométhyl-1-[(3-fluorophényl)méthyl]-1*H*-indole-2-carboxamide,
*N*-(isoquinoléin-6-yl)-5-fluoro-1-[(3-fluorophényl)méthyl]-1*H*-indole-2-carboxamide,
*N*-(1-méthyl-benzimidazol-5-yl)-5-fluoro-1-[(3-fluorophényl)méthyl]-1*H*-indole-2-carboxamide,
*N*-(1-méthyl-benzimidazol-4-yl)-5-fluoro-1-[(3-fluorophényl)méthyl]-1*H*-indole-2-carboxamide,
*N*-(1*H*-benzotriazol-5-yl)-5-trifluorométhyl-1-[(3-fluorophényl)méthyl]-1*H*-indole-2-carboxamide,
*N*-(quinoléin-6-yl)-5-fluoro-1-[(3-fluorophényl)méthyl]-1*H*-indole-2-carboxamide,
*N*-(1-méthyl-indazol-5-yl)-5-fluoro-1-[(3-fluorophényl)méthyl]-1*H*-indole-2-carboxamide,
*N*-(2-méthyl-benzoxazol-5-yl)-5-fluoro-1-[(3-fluorophényl)méthyl]-1*H*-indole-2-carboxamide,
*N*-(benzothiazol-6-yl)-5-fluoro-1-[(3-fluorophényl)méthyl]-1*H*-indole-2-carboxamide,
*N*-(2-méthyl-benzothiazol-5-yl)-5-fluoro-1-[(3-fluorophényl)méthyl]-1*H*-indole-2-carboxamide,
*N*-(2-oxo-3,4-dihydroquinoléin-7-yl)-5-trifluorométhyl-1-[(3-fluorophényl)méthyl]-1*H*-indole-2-carboxamide,
*N*-(2-oxo-indolin-5-yl)-5-fluoro-1-[(3-fluorophényl)méthyl]-1*H*-indole-2-carboxamide,
*N*-(1*H*-benzotriazol-5-yl)-5-fluoro-1-[(3-fluorophényl)méthyl]-1*H*-indole-2-carboxamide,
*N*-(1-méthylsulfonyl-indolin-5-yl)-5-fluoro-1-[(3-fluorophényl)méthyl]-1*H*-indole-2-carboxamide,
*N*-(1,2-diméthyl-benzimidazol-5-yl)-5-fluoro-1-[(3-fluorophényl)méthyl]-1*H*-indole-2-carboxamide,
*N*-(2-éthyl-benzoxazol-5-yl)-5-trifluorométhyl-1-[(3-fluorophényl)méthyl]-1*H*-indole-2-carboxamide,
*N*-(2-phényl-benzoxazol-5-yl)-5-tifluorométhyl-1-[(3-fluorophényl)méthyl]-1*H*-indole-2-carboxamide,
*N*-(quinoxalin-6-yl)-5-fluoro-1-[(3-fluorophényl)méthyl]-1*H*-indole-2-carboxamide,
*N*-(quinoléin-7-yl)-5-fluoro-1-[(3-fluorophényl)méthyl]-1*H*-indole-2-carboxamide,
*N*-(isoquinoiéin-7-yl)-5-fluoro-1-[(3-fluorophényl)méthyl]-1*H*-indole-2-carboxamide,
*N*-(2-méthylbenzimidazol-5-yl)-5-fluoro-1-[(3-fluorophényl)méthyl]-1*H*-indole-2-carboxamide,
*N*-(benzimidazol-5-yl)-5-fluoro-1-[(3-fluorophényl)méthyl]-1*H*-indole-2-carboxamide,
*N*-(2-oxo-3,4-dihydroquinoléin-7-yl)-6-méthoxy-1-[(4-fluorophényl)méthyl]-1*H*-indole-2-carboxamide et
*N*-(1-méthylbenzimidazol-6-yl)-5-fluoro-1-[(3-fluorophényl)méthyl]-1*H*-indole-2-carboxamide.

Dans le cadre de la présente invention on entend par :

- $C_t$-$C_z$ où t et z peuvent prendre les valeurs de 1 à 7, une chaîne carbonée pouvant avoir de t à z atomes de carbone, par exemple $C_1$-$C_3$ une chaîne carbonée qui peut avoir de 1 à 3 atomes de carbone;
- un alkyle : un groupe aliphatique saturé linéaire ou ramifié. A titre d'exemples, on peut citer les groupes méthyle, éthyle, propyle, isopropyle, butyle, isobutyle, tertiobutyle, pentyle, etc ;
- un alkylène : un groupe alkyle divalent saturé, linéaire ou ramifié, par exemple un groupe $C_{1-3}$-alkylène représente une chaîne carbonée divalente de 1 à 3 atomes de carbone, linéaire ou ramifiée, plus particulièrement un méthylène, éthylène, 1-méthyléthylène, propylène;
- un cycloalkyle : un groupe carboné cyclique. A titre d'exemples, on peut citer les groupes cyclopropyle, cyclobutyle, cyclopentyle, cyclohexyle, etc ;
- un fluoroalkyle : un groupe alkyle dont un ou plusieurs atomes d'hydrogène ont été substitués par un atome de fluor ;
- un alcoxy : un radical -O-alkyle où le groupe alkyle est tel que précédemment défini ;
- un fluoroalcoxy : un groupe alcoxy dont un ou plusieurs atomes d'hydrogène ont été substitués par un atome de fluor ;
- un thioalkyle: un radical -S-alkyle où le groupe alkyle est tel que précédemment défini ;
- un aryle : un groupe aromatique cyclique comprenant entre 6 et 10 atomes de carbones. A titre d'exemples de groupes aryles, on peut citer les groupes phényle ou naphtyle ;
- un hétérocycle: un groupe cyclique saturé, partiellement insaturé ou aromatique de 5 à 7 chaînons, comprenant de un à trois hétéroatomes choisi parmi O, S ou N. A titre d'exemples de groupe W, on peut citer les groupes indolinyle, isoindolinyle, benzofuranyle, dihydrobenzofuranyle, benzothiophényle, dihydrobenzothiophényle, benzoxazolyle, dihydrobenzoxazolinyle, isobenzofuranyle, dihydroisobenzofuranyle, benzimidazolyle, dihydrobenzimidazolyle, indazolyle, benzothiazolyle, isobenzothiazolyle, dihydroisobenzothiazolyle, benzotriazolyle, quinoléinyle, dihydroquinoléinyle, tétrahydroquinoléinyle, isoquinoléinyle, dihydroisoquinoléinyle, tétrahydroisoquinoléinyle, benzoxazinyle, dihydrobenzoxazinyle, benzothiazinyle, dihydrobenzothiazinyle, cinnolinyle, quinazolinyle, dihydroquinazolinyle, tétrahydroquinazolinyle, quinoxalinyle, dihydroquinoxalinyle, tétrahydroquinoxalinyle, phtalazinyle, dihydrophtalazinyle, tétrahydrophtalazinyle, tétrahydrobenz[*b*]azépinyle, tétrahydrobenz[*c*]azépinyle, tétrahydrobenz[d]azépinyle, tétrahydrobenz[d]azépinyle, tétrahydrobenzo[b][1,4]diazépinyle, tétrahydrobenzo[e][1,4]diazépinyle, tétrahydrobenzo[b][1,4]oxazépinyle ou tétrahydrobenzo[b][1,4]thiazépinyle ;
- un atome d'halogène: un fluor, un chlore, un brome ou un iode ;
- « oxo » signifie « =O » ;

- « thio » signifie « =S ».

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.

Dans ce qui suit, on entend par groupe partant, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans « Advances in Organic Chemistry », J. March, 5th Edition, Wiley Interscience, 2001.

Conformément à l'invention on peut préparer les composés de formule générale (I) selon le procédé illustré par le schéma 1 qui suit.

Selon le schéma 1, les composés de formule générale (IV) peuvent être obtenus par réaction d'un composé de formule générale (II) dans laquelle $X_1$, $X_2$, $X_3$, $X_4$ sont tels que définis dans la formule générale (I) ci-dessus et B représente un groupe $C_1$-$C_6$-alcoxy ou hydroxy, avec un composé de formule générale (III), dans laquelle $Z_1$, $Z_2$, $Z_3$, $Z_4$, $Z_5$ et n sont tels que définis dans la formule générale (I) ci-dessus et R' représente un atome de brome, d'iode, un groupe tosylate ou tout autre groupe partant.

Lorsque n = 1, 2 ou 3, le composé de formule générale (III) peut être un halogénure d'alkyle, tel qu'un bromure de benzyle (n =1 : Kolasa T., Bioorg.Med.Chem. 1997, 5 (3) 507) ou un iodure de phénéthyle (n = 2 : Abramovitch R., Synth. Commun., 1995, 25 (1), 1), et la réaction peut être réalisée en présence d'une base telle que l'hydrure de sodium ou le carbonate de potassium, dans un solvant polaire tel que le diméthylformamide, le diméthylsulfoxyde ou l'acétone.

Schéma 1

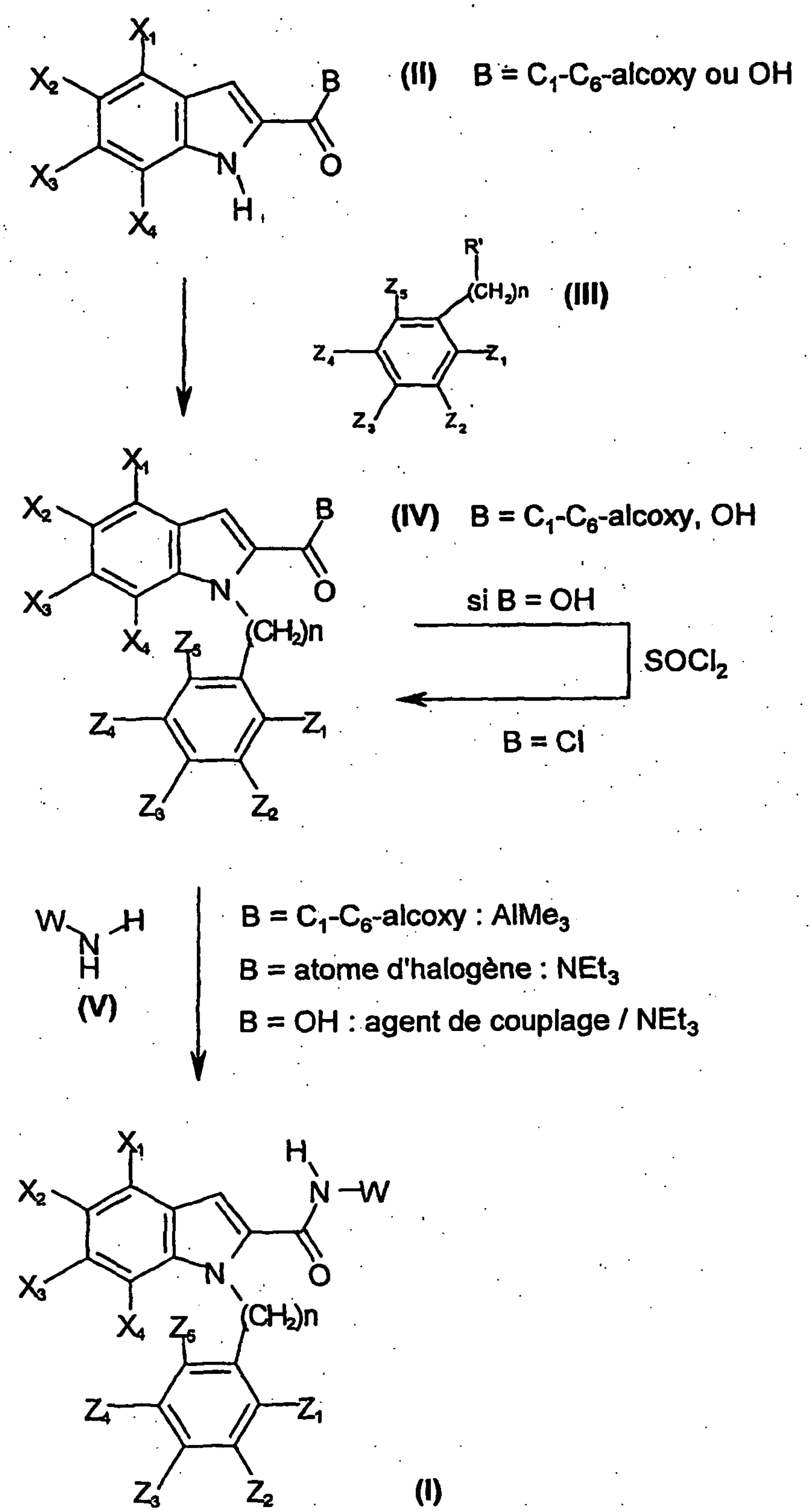


Lorsque n = 0, le composé de formule générale (III) est un iodure ou un bromure d'aryle et la réaction peut

être réalisée à une température comprise entre 80˚C et 250˚C, en présence d'un catalyseur à base de cuivre tel que le bromure de cuivre ou l'oxyde de cuivre ainsi que d'une base telle que le carbonate de potassium (Murakami Y., Chem.Pharm.Bull., 1995, 43 (8), 1281). On peut également utiliser les conditions plus douces, décrites dans S.L. Buchwald, J.Am.Chem.Soc. 2002, 124, 11684. Alternativement, les composés de formule générale (IV), dans laquelle n = 0, peuvent être obtenus par réaction du composé de formule générale (II) avec un composé de formule générale (III) de type acide boronique (n = 0, R' = $B(OH)_2$), en présence d'une base telle que la triéthylamine ou la pyridine ainsi que de diacétate de cuivre, par analogie avec des protocoles décrits dans W.W.K.R. Mederski, Tetrahedron, 1999, 55, 12757.

Les composés de formule générale (II) sont disponibles dans le commerce ou préparés selon de nombreux procédés décrits dans la littérature (D. Knittel Synthesis 1985, 2, 186 ; T.M. Williams J.Med.Chem. 1993, 36 (9), 1291 ; JP2001151771A2 par exemple).

Dans le cas des indoles de formule générale (IV), dans laquelle B représente un groupe $C_1$-$C_6$-alcoxy, le composé de formule générale (I) est obtenu par réaction d'un composé de formule générale (IV), tel qu'obtenu ci-dessus, avec un amidure du composé de formule générale (V), dans laquelle W est tel que défini dans la formule générale (I) ci-dessus, au reflux d'un solvant tel que le toluène. L'amidure du composé de formule générale (V) est préparé par action préalable du triméthylaluminium sur les amines de formule générale (V).

Dans le cas des indoles de formule générale (IV), dans laquelle B représente un groupe hydroxy, la fonction acide carboxylique peut préalablement être transformée en halogénure d'acide tel qu'un chlorure d'acide par action du chlorure de thionyle, au reflux d'un solvant tel que le dichlorométhane ou le dichloroéthane. Le composé de formule générale (I) est alors obtenu par réaction du composé de formule générale (IV), dans laquelle B représente un atome de chlore, avec le composé de formule générale (V), en présence d'une base telle que la triéthylamine ou le carbonate de sodium. Alternativement, l'indole de formule générale (IV), dans laquelle B représente un groupe hydroxy, peut être couplé avec le composé de formule générale (V) en présence d'un agent de couplage tel qu'un dialkylcarbodiimide, l'hexafluorophosphate de benzotriazole-1-yl-oxy-trispyrrolidinophosphonium, le diéthylcyanophosphonate ou tout autre agent de couplage connu de l'homme de l'art, en présence d'une base comme la triéthylamine, dans un solvant tel que le diméthylformamide.

Dans le schéma 1, les composés de formule (II), (III) et (V) et les autres réactifs, quand leur mode de préparation n'est pas décrit, sont disponibles dans le commerce ou décrits dans la littérature (WO2003049702 WO2003068749 par exemple).

Les composés de formules générales (II), (IV) et (I), dans lesquelles $X_1$, $X_2$, $X_3$, $X_4$, $Z_1$, $Z_2$, $Z_3$, $Z_4$ et/ou $Z_5$ représentent un groupe cyano ou un aryle, peuvent être obtenus par une réaction de couplage, catalysée par un métal tel que le palladium, réalisée sur les composés de formules générales (II), (IV) ou (I) correspondants, dans lesquelles $X_1$, $X_2$, $X_3$, $X_4$, $Z_1$, $Z_2$, $Z_3$, $Z_4$ et/ou $Z_5$ représente un atome de brome.

Les composés de formules générales (II), (IV) et (I), dans lesquelles $X_1$, $X_2$, $X_3$, $X_4$, $Z_1$, $Z_2$, $Z_3$, $Z_4$et/ou $Z_5$ représentent un groupe $C(O)NR_1R_2$, peuvent être obtenus à partir des composés de formules générales (II), (IV) ou (I) correspondants, dans lesquelles $X_1$, $X_2$, $X_3$, $X_4$, $Z_1$, $Z_2$, $Z_3$, $Z_4$ et/ou $Z_5$ représente un groupe cyano, selon des méthodes qui sont décrites dans la littérature ou qui sont connues de l'homme du métier.

Les composés de formules générales (II), (IV) et (I), dans lesquelles $X_1$, $X_2$, $X_3$, $X_4$, $Z_1$, $Z_2$, $Z_3$, $Z_4$ et/ou $Z_5$ représentent un groupe -S(O)-alkyle ou $-S(O)_2$-alkyle, peuvent être obtenus par oxydation des composés de formules générales (II), (IV) ou (I) correspondants, dans lesquelles $X_1$, $X_2$, $X_3$, $X_4$, $Z_1$, $Z_2$, $Z_3$, $Z_4$ et/ou $Z_5$ représente un groupe $C_1$-$C_6$-thioalkyle, selon des méthodes qui sont décrites dans la littérature ou qui sont connues de l'homme du métier.

Les composés de formules générales (II), (IV) et (I), dans lesquelles $X_1$, $X_2$, $X_3$, $X_4$, $Z_1$, $Z_2$, $Z_3$, $Z_4$ et/ou $Z_5$ représentent un groupe $NR_1R_2$, $NR_3COR_4$ ou $NR_3SO_2R_5$, peuvent être obtenus à partir des composés de formules générales (II), (IV) ou (I) correspondants, dans lesquelles $X_1$, $X_2$, $X_3$, $X_4$, $Z_1$, $Z_2$, $Z_3$, $Z_4$ et/ou $Z_5$ représente un groupe nitro, par exemple par réduction, puis acylation ou sulfonylation, selon des méthodes qui sont décrites dans la littérature ou qui sont connues de l'homme du métier.

Les composés de formules générales (II), (IV) et (I), dans lesquelles $X_1$, $X_2$, $X_3$, $X_4$, $Z_1$, $Z_2$, $Z_3$, $Z_4$ et/ou $Z_5$ représentent un groupe $SO_2NR_1R_2$ peuvent être obtenus par une méthode analogue à celle décrite dans Pharmazie 1990, 45, 346, ou selon des méthodes qui sont décrites dans la littérature ou qui sont connues de l'homme du métier.

Les composés de formule générale (I), dans laquelle $R_7$ représente un atome d'hydrogène peuvent être obtenus à partir de composés de formule générale (I) dans laquelle $R_7$ représente un groupe phénylméthyle, par hydrogénation catalysée, par exemple par le palladium, ou par toutes méthodes qui sont décrites dans la littérature ou qui sont connues de l'homme du métier.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau 1. Les microanalyses élémentaires, les analyses LC-MS (chromatographie liquide couplée à la spectrométrie de masse) les spectres I.R. et R.M.N. confirment les structures des composés obtenus.

**Exemple 1 (Composé N°12)**

*N*-(1-méthyl-1*H*-indolin-5-yl)-5-fluoro-1-[(3-fluorophényl)méthyl]-1*H*-indole-2-carboxamide

1.1. 5-fluoro-1-[(3-fluorophényl)méthyl]-1*H*-indole-2-carboxylate d'éthyle On agite une suspension de 0,207 g (1 mmole) de 5-fluoro-1*H*-indole-2-carboxylate d'éthyle, de 0,173 g (1,2 mmole) de chlorure de 3-fluorobenzyle et de 0,276 g (2 mmoles) de carbonate de potassium dans 10 mL de diméthylformamide pendant 24 heures à 60°C. On refroidit ensuite le mélange réactionnel, on le verse dans un mélange d'eau glacée et d'acétate d'éthyle. Après décantation, on sépare la phase organique puis on la lave avec deux fois 50 mL d'eau puis avec 50 mL d'une solution saturée en chlorure > de sodium. On sèche la solution sur sulfate de magnésium, on la filtre puis on concentre le filtrat sous pression réduite. On obtient 0,195 g d'une huile utilisée telle quelle dans l'étape suivante.

1.2 *N*-(1-méthyl-1*H*-indolin-5-yl)-5-fluoro-1-[(3-fluorophényl)méthyl]-1*H*-indole-2-carboxamide (composé n°12)

On ajoute sous argon 0,089 g (0,6 mmole) de 5-amino-1-méthyl-1*H*-indoline (WO2003049702) et 0,5 mL de triméthylaluminium (2M dans le toluène) dans 2 mL de toluène. On chauffe 2h à 50°C et on ajoute 0,157 g (0,5 mmole) de 5-fluoro-1-[(3-fluorophényl)méthyl]-1*H*-indole-2-carboxylate d'éthyle, obtenu à l'étape 1.1. On porte à reflux pendant 10 mn et on laisse le milieu réactionnel à température ambiante toute la nuit. On le verse sur de la glace et on ajoute 1 mL d'acide chlorhydrique 1 N. On extrait par de l'acétate d'éthyle, on sèche la phase organique par du sulfate de magnésium et on la concentre sous pression réduite. On purifie le résidu par chromatographie préparative. On obtient 0,066 g de solide.
Point de fusion : 145-147 °C
R.M.N. $^1$H (DMSO $D_6$), δ (ppm) : 2,65 (s, 3H) ; 2,85 (t, 2H) ; 3,2 (t, 2H) ; 5,85 (s, 2H) ; 6,45 (d, 1H) ; 6,9 (m, 2H) ; 7,1 (m, 2H) ; 7,3 (m, 3H) ; 7,5 (m, 3H) ;

**Exemple 2 (composé n°13)**

*N*-(1-méthyl-1,2,3,4-tétrahydroquinoléin-7-yl)-5-fluoro-1-[(3-fluorophényl)méthyl]-1*H*-indole-2-carboxamide

On procède selon la méthode décrite à l'étape 1.2 de l'exemple 1 à partir de 0,185 g de 7-amino-1-méthyl-1,2,3,4-tétrahydroquinoléine (WO2003049702), de 0,95 mL de triméthylaluminium (2M dans le toluène) et de 0,3 g de 5-fluoro-1-[(3-fluorophényl)méthyl]-1*H*-indole-2-carboxylate d'éthyle, obtenu à l'étape 1.1 de l'exemple 1. On obtient 0,122 g de produit.
Point de fusion : 159 - 1160 °C
R.M.N. $^1$H (DMSO $D_6$) : δ (ppm) : 1,85 (m, 2H) ; 2,65 (t, 2H) ; 2,8 (s. 3H) ; 3,15 (t, 2H) : 5,85 (s, 2H) ; 7 (m, 7H) ; 7,3 (m, 2H) ; 7,5 (m, 2H) ; 10,1 (s, 1H)

**Exemple 3 (composé n°14)**

*N*-(1,2,3,4-tétrahydroquinoléin-7-yl)-5-fluoro-1-[(3-fluorophényl)méthyl]-1*H*-indole-2-carboxamide

On procède selon la méthode décrite à l'étape 1.2 de l'exemple 1 à partir de 0,169 g de 7-amino-1,2,3,4-tétrahydroquinoléine (WO2003049702), de 0,95 mL de triméthylaluminium (2M dans le toluène) et de 0,3 g de 5-fluoro-1-[(3-fluorophényl)méthyl]-1*H*-indole-2-carboxylate d'éthyle, obtenu à l'étape 1.1 de l'exemple 1. On obtient 0,033 g de produit.
Point de fusion: 149 - 151 °C
R.M.N. $^1$H (DMSO $D_6$) : δ (ppm) : 1,75 (m, 2H) ; 2,6 (t, 2H) ; 3,1 (t, 2H) ; 5,85 (s, 2H) ; 6,95 (m, 7H) ; 7,3 (m, 2H) ; 7,5 (m, 2H) ; 10,1 (s, 1H)

**Exemple 4 (composé n°18)**

*N*-(2-méthyl-benzoxazol-5-yl)-1-[3-(trifluorométhyl)phényl]-1*H*-indole-2-carboxamide

On ajoute 0,091 mL (0,6 mmole) de diéthylcyanophosphonate, 0,168 mL (0,6 mmole) de triéthylamine et 0,111g (0,6 mmole) de chlorhydrate de 5-amino-2-méthyl-benzoxazole à une solution de 0,152 g (0,5 mmole) d'acide 1-[3-(trifluorométhyl)phényl]-1*H*-indole-2-carboxylique (JP2001151771A2) dans 3 mL de diméthylformamide. On agite une nuit à température ambiante, on concentre sous pression réduite et on reprend le résidu par de l'eau et du dichlorométhane. Après décantation, la phase organique est séchée et évaporée sous pression réduite. On purifie le résidu par chromatographie préparative. On obtient 0,102 g de solide.
Point de fusion : 223-225 °C
R.M.N. $^1$H (DMSO $O_6$) : δ (ppm) : 2,55 (s, 3H) ; 7,2 (m, 3H) ; 7,6 (m, 3H) ; 7,75 (m, 5H) ; 7,95 (s, 1H) ; 10,5 (s, 1H)

**Exemple 5 (composé n°19)**

*N*-(1-méthyl-1*H*-indazol-5-yl)-5-trifluorométhyl-1-[(3-fluorophényl)méthyl]-1*H*-indole-2-carboxamide

5.1 5-trifluorométhyl-1-[(3-fluorophényl)méthyl]-1*H*-indole-2-carboxylate d'éthyle

On ajoute goutte à goutte une solution de 2,88 g (11,2 mmoles) de 5-trifluorométhyl-1*H*-indole-2-carboxylate d'éthyle (*obtenu par synthèse indolique de Fisher à partir de la* 4-(trifluorométhyl)phénylhydrazine) dans 50 ml de diméthylformamide dans une suspension de 0,58 g (14,56 mmoles) d'hydrure de sodium dans 5 ml de diméthylformamide refroidi par un bain de glace. On agite 2h à température ambiante puis on ajoute une solution de 2,54 g (13,44 mmoles) de bromure de 3-fluorobenzyle dans 20 ml de diméthylformamide. On poursuit l'agitation pendant 24h. On rajoute 2,44 moles de bromure de 3-fluorobenzyle et on laisse agiter pendant 4h supplémentaires. On évapore sous pression réduite le solvant et on reprend le résidu par de l'eau et de l'acétate d'éthyle. Après décantation, on sépare la phase organique puis on la lave avec deux fois 50 mL d'eau puis avec 50 mL d'une solution saturée en chlorure de sodium. On sèche la solution sur sulfate de magnésium, on la filtre puis on concentre le filtrat sous pression réduite. On purifie le résidu par chromatographie sur gel de silice. On obtient 2,74 g de produit.

5.2. *N*-(1-méthyl-1*H*-indazol-5-yl)-5-trifluorométhyl-1-[(3-fluorophényl)méthyl)-1*H*-indole-2-carboxamide

On ajoute, sur bain de glace, une solution de 0,34 g (2,3 moles) de 5-amino-1-méthyl-1*H*-indazole (I.T. Forbes, J. Med. Chem. 1993, 36 (8), 1104) dans 10 mL de toluène à une solution de 1,92 mL (3,83 mmoles) de triméthylaluminium (2M dans le toluène) dans 5 mL de toluène. On porte le milieu réactionnel à 50°C pendant 30 mn. On additionne ensuite (1,92 mmole) de 5-trifluorométhyl-1-[(3-fluorophényl)méthyl]-1*H*-indole-2-carboxylate d'éthyle, obtenu à l'étape 5.1, en solution dans 10 mL de toluène. On chauffe le mélange pendant 3h à reflux et on le laisse revenir à température ambiante. On ajoute 20 mL d'eau et 30 mL d'acétate d'éthyle. On extrait la phase aqueuse par de l'acétate d'éthyle ; on rassemble les phases organiques que l'on lave par de l'eau puis par une solution saturée en chlorure de sodium. On sèche la solution sur sulfate de magnésium, on la filtre puis on concentre le filtrat sous pression réduite. On purifie le résidu par chromatographie sur colonne de silice en éluant avec un mélange d'acétate d'éthyle et de dichlorométhane. On reprend le résidu par de l'éther de pétrole, on le filtre, on le rince et on le sèche sous pression réduite. On obtient 0,71 g de solide.
Point de fusion : 198 -199,°C
R.M.N. $^1$H ($CDCl_3$): δ (ppm) : 4 (s, 3H) ; 5,9 (s, 2H) ; 6,9 (m, 2H) ; 7 (m, 1H) ; 7,3 (m, 1H) ; 7,6 (m, 4H) ; 7,8 (d, 1H) ; 8 (s, 1H) ; 8,2 (d, 2H) ; 10,6 (s, 1H).

**Exemple 6 (composé n°20)**

*N*-(1*H*-2-oxo-3,4-dihydroquinoléin-7-yl)-5-fluoro-1-[(3-fluorophényl)méthyl]-1*H*-indole-2-carboxamide

On ajoute sous argon 0,097 g (0,6 mmole) de 7-amino-1*H*-3,4-dihydroquinoléin-2-one (WO2003049702) et 0,5 mL de triméthylaluminium (2M dans le toluène) dans 2 mL de toluène. On chauffe 2h à 50°C et on ajoute 0,157 g (0,5 mmole) de 5-fluoro-1-[(3-fluorophényl)méthyl]-1*H*-indole-2-carboxylate d'éthyle, obtenu à l'étape 1.1 de l'exemple 1, en solution dans 1 mL de toluène. On porte à reflux pendant 2h et on laisse le milieu réactionnel à température ambiante toute la nuit. On le verse sur de la glace et on ajoute 2 mL d'acide chlorhydrique 1 N. On extrait par de l'acétate d'étyle, on sèche la phase organique par du sulfate de magnésium et on la concentre sous pression réduite. On purifie le résidu par chromatographie préparative. On obtient 0,047 g de solide.
Point de fusion : 277- 279 °C
R.M.N. $^1$H (DMSO $D_6$) : δ (ppm) : 2,4 (t, 2H) ; 2,8 (t, 2H) ; 5,85 (s, 2H) ; 6,9 (m, 2H) ; 7,1 (m, 5H) ; 7,4 (m, 2H) ; 7,5 (m, 2H) ; 10,05 (s, 1H) ; 10,4 (s, 1H)

**Exemple 7 (composé n°22)**

*N*-(2,3-dihydrobenzoxazin-6-yl)-5-fluoro-1-[(3-fluorophényl)méthyl]-1*H*-indole-2-carboxamide

On procède selon la méthode décrite dans l'exemple 6 à partir de 0,090 g de 6-amino-2,3-dihydrobenzoxazine (WO2003049702), de 0,5 mL de triméthylaluminium (2M dans le toluène) et de 0,157 g de 5-fluoro-1-[(3-fluorophénynméthyl)-1H-indole-2-carboxylate d'éthyle, obtenu à l'étape 1.1 de l'exemple 1. On obtient 0,061 g de produit Point de fusion : 216-217 °C
R.M.N. $^1$H (DMSO $D_6$) : δ (ppm) : 3,25 (t, 2H) ; 4,1 (t, 2H) ; 5,85 (s, 2H+1H) ; 6,55 (d, 1H) ; 6,9 (m, 3H) ; 7,1 (m, 3H) ; 7,3 (m, 2H) ; 7,5 (m, 2H) ; 10,1 (s, 1H)

**Exemple 8 (composé n°23)**

*N*-(3-oxo-2*H*-benzoxazin-6-yl)-5-fluoro-1-[(3-fluorophényl)méthyl]-1*H*-indole-2-carboxamide

On procède selon la méthode décrite dans l'exemple 6 à partir de 0,107 g de 6-amino-3-oxo-2*H*-benzoxazine (WO2003049702), de 0,5 mL de triméthylaluminium (2M dans le toluène) et de 0,157 g de 5-fluoro-1-[(3-fluorophényl) méthyl]-1*H*-indole-2-carboxylate d'éthyle, obtenu à l'étape 1.1 de l'exemple 1. On obtient 0,053 g de produit.
LC-MS : M+H = 434
R.M.N. $^1$H (DMSO $D_6$) δ (ppm) : 4,5 (s, 2H) ; 5,85 (s, 2H) ; 6,9 (m, 3H) ; 7,1 (m, 4H) ; 7,4 (s, 1H) ; 7,5 (m, 3H) ; 10,4 (s, 1H) ; 10,7 (s, 1H)

**Exemple 9 (composé n°24)**

*N*-(1,2,3,4-tétrahydroquinoléin-7-yl)-5-trifluorométhyl-1-[(3-fluorophényl)méthyl]-1*H*-indole-2-carboxamide

On ajoute, à 0°C sur bain de glace, une solution de 0,24 g (1,64 mmole) de 7-amino-1,2,3,4-tétrahydroquinoléine (WO2003049702) dans 5 mL de toluène à une solution de 1,37 mL (2,74 mmoles) de triméthylaluminium (2M dans le toluène) dans 5 mL de toluène. On porte le milieu réactionnel à 50°C pendant 2h. On additionne ensuite 0,5 g (1,37 mmole) de 5-trifluorométhyl-1-[(3-fluorophényl)méthyl]-1*H*-indole-2-carboxylate d'éthyle, obtenu à l'étape 5.1 de l'exemple 5, en solution dans 10 mL de toluène. On chauffe le mélange pendant 3h à reflux et on le laisse revenir à température ambiante. On ajoute 20 mL d'eau glacée, 20 mL d'acétate d'éthyle et 20 ml d'acide chlorhydrique 1N. Après filtration du mélange et décantation, on lave la phase organique par une solution alcaline puis par une solution saturée en chlorure de sodium. On sèche la phase organique sur sulfate de magnésium, on la filtre puis on concentre le filtrat sous pression réduite. On le reprend par de l'éther de pétrole, on recueille le résidu par filtration et on le sèche sous pression réduite. On le purifie par chromatographie sur colonne de silice en éluant avec un mélange d'heptane et de dichlorométhane. On recristallise le résidu dans l'éthanol. On obtient 0,29 g de solide.
Point de fusion : 203 - 204 °C
R.M.N. $^1$H (DMSO) : δ (ppm) : 1,7 (m, 2H) ; 2,6 (m, 2H) ; 3,1 (m, 2H) ; 5,7 (t, 1H) ; 5,9 (s, 2H) ; 6,7 (m, 2H) ; 6,95 (m, 4H) ; 7,3 (m, 1H) ; 7,45 (s, 1H) ; 7,5 (d, 1H) ; 7,75 (d, 1H) ; 8,1 (s, 1H) ; 10,2 (s, 1H).

**Exemple 10 (composé n°26)**

*N*-(1-méthyl-benzimidazol-5-yl)-5-trifluorométhyl-1-[(3-fluorophényl)méthyl]-1*H*-indole-2-carboxamide

10.1 Acide 5-trifluorométhyl-1-[(3-fluorophényl)méthyl]-1*H*-indole-2-carboxylique

On porte à reflux une solution de 0,7 g (1,92 mole) de 5-trifluorométhyl-1-[(3-fluorophényl)méthyl]-1*H*-indole-2-carboxylate d'éthyle, obtenu à l'étape 5.1 de l'exemple 5, et de 0,21 g (3,83 mmoles) d'hydroxyde de potassium dans 10 mL de méthanol. On concentre sous pression réduite; on reprend le résidu par de l'eau et on acidifie par de l'acide chlorhydrique. On recueille le précipité par filtration, on le rince à l'eau et on le sèche sous pression réduite. On obtient 0,69 g de solide que l'on utilise tel quel dans l'étape suivante.

10.2 *N*-(1-méthyl-benzimidazol-5-yl)-5-trifluorométhyl-1-[(3-fluorophényl)méthyl]-1H-indole-2-carboxamide

On porte à reflux pendant 2h une solution de 0,32 g (0,95 mmole) d'acide 5-trifluorométhyl-1-[(3-fluorophényl) méthyl]-1*H*-indole-2-carboxylique, obtenu à l'étape 10.1, et de 0,69 mL (9,49 mmoles) de chlorure de thionyle dans 25 mL de dichlorométhane. On concentre sous pression réduite, on reprend le résidu par 20 mL d'éther diéthylique et on additionne 0,17 g (1,14 mmole) de 5-amino-1-méthyl-benzimidazole et une solution de 0,2 g (1,9 mmole) de carbonate de sodium dans 2 mL d'eau. On agite pendant 24h à température ambiante, on évapore la phase organique sous pression réduite et on extrait la phase résultante par de l'acétate d'éthyle et du dichlorométhane. Les phases organiques sont lavées à l'eau et par une solution saturée de chlorure de sodium, séchées sur sulfate de sodium et concentrées sous pression réduite. On reprend le résidu par de l'éther de pétrole, on le recueille par filtration, on le lave et on le sèche sous pression réduite. Il est ensuite purifié par chromatographie sur colonne de gel de silice en éluant avec un

mélange de dichlorométhane et d'acétate d'éthyle. On reprend le résidu par de l'éther de pétrole, on le recueille par filtration, on le lave et on le sèche sous pression réduite. On obtient 0,3 g de solide.
Point de fusion : 223 - 224°C
R.M.N. $^1$H (DMSO), δ (ppm) : 5,9 (s, 2H) ; 7 (m, 3H) ; 7,3 (m, 1H) ; 7,55 (m, 4H) ; 7,8 (d, 1H) ; 8,05 (s, 1H) ; 8,1 (d, 2H) ; 10,5 (s, 1H).

**Exemple 11 (composé n°49)**

*N*-(quinoléin-7-yl)-5-fluoro-1-[(3-fluorophényl)-méthyl]-1*H*-indole-2-carboxamide

11.1 Acide 5-fluoro-1-[(3-fluorophényl)méthyl]-1*H*-indole-2-carboxylique

On porte à reflux pendant 2h une solution de 8,3 g (26.3 mmoles) de 5-fluoro-1-[(3-fluorophényl)méthyl]-1*H*-indole-2-carboxylate d'éthyle, obtenu à l'étape 1.1 de l'exemple 1, et de 5,2 g (79 mmoles) d'hydroxyde de potassium dans une solution de 140 mL d'éthanol et de 14 mL d'eau. On concentre sous pression réduite, on reprend le résidu par de l'eau et on acidifie par de l'acide chlorhydrique. On recueille le précipité par filtration, on le rince à l'eau et on le sèche sous pression réduite. On obtient 7,4 g de solide que l'on utilise tel quel dans l'étape suivante.
Point de fusion : 205 - 206°C

11.2 *N*-(quinoléin-7-yl)-5-fluoro-1-[(3-fluorophényl)méthyl]-1*H*-indole-2-carboxamide

On ajoute, sous agitation et sous azote, 1 g (1,9 mmole) d'hexafluorophosphate de (benzotriazol-1-yl-oxy) trispyrrolidinophosphonium à une suspension de 0,5 g (1,74 mmole) d'acide 5-fluoro-1-[(3-fluorophényl)méthyl]-1*H*-indole-2-carboxylique, obtenu à l'étape 11.1, dans 10 mL de diméthylformamide sec. Après 5 mn, on ajoute 0,4 g (1,83 mmole) de chlorhydrate de 7-aminoquinoléine (WO2003049702) et 0,9 g (7 mmoles) de diisopropyléthylamine. Après 2h d'agitation à température ambiante et 2h à 60°C, on verse le milieu réactionnel sur 100 mL d'eau et 50 mL d'acétate d'éthyle. Après décantation et extraction de la phase aqueuse, les phases organiques sont rassemblées, lavées à l'eau et séchées sur sulfate de sodium. On purifie le résidu par chromatographie sur colonne de gel de silice en éluant avec un mélange de dichlorométhane et d'acétone. On recristallise le solide obtenu dans l'alcool isopropylique. On obtient 0,26 g de solide.
Point de fusion : 222-223°C
R.M.N. $^1$H (DMSO), δ (ppm) : 5,95 (s, 2H) ; 6,95 (t, 2H) ; 7,05 (m, 1H) ; 7,2 (t, 1H) ; 7,35 (q, 1H) ; 7,45 (m, 1H) ; 7,55 (s, 1H) ; 7,65 (m, 2H) ;8 (s, 2H) ; 8,3 (d, 1H) ; 8,55 (s, 1H) ; 8,9 (m, 1H) ; 11 (s, 1H).

Le tableau 1 qui suit illustre les structures chimiques et les propriétés physiques de quelques composés de formule générale (I) selon l'invention. Dans ce tableau, la colonne "PF" renseigne les points de fusion des produits en degrés Celsius (°C). Lorsque les produits ont été isolés sous la forme de solide amorphe ou d'huile, ils sont caractérisés dans cette colonne par leur masse ([MH]$^+$). D'autre part, dans la colonne « sel », « - » représente un composé sous forme de base libre, alors que « HCl » représente un composé sous forme de chlorhydrate et le rapport entre parenthèses est le rapport (acide : base).

Tableau 1

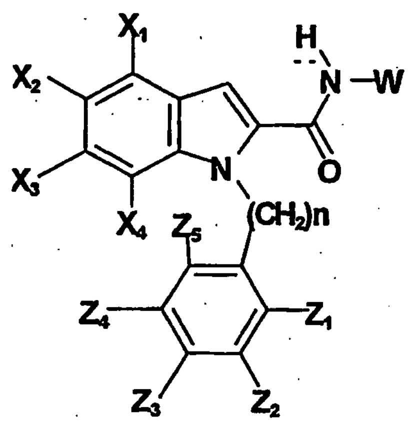

(I)

(suite)

| N° | $X_1$, $X_2$, $X_3$, $X_4$ | n | $Z_1$, $Z_2$, $Z_3$, $Z_4$, $Z_5$ | W | PF (°C) | Sel |
|---|---|---|---|---|---|---|
| 1 | H, F, H, H | 1 | H, $CF_3$, H, H, H | isoquinoléin-5-yle | 208 - 209 | - |
| 2 | H, H, H, H | 0 | H, $CF_3$, H, H, H | 1-méthyl-1,2,3,4-tétrahydroquinoléin-7-yle | 92-95 | - |
| 3 | H, H, H, H | 0 | H, $CH_3$, H, $CH_3$, H | 1-méthyl-1,2,3,4-tétrahydroquinoléin-7-yle | 176-178 | - |
| 4 | H, H, H, H | 0 | H, $CF_3$, H, H, H | 1,2,3,4-tétrahydroquinoléin-7-yle | 140-143 | - |
| 5 | H, H, H, H | 0 | H, $CF_3$, H, H, H | 4-méthyl-3-oxo-2*H*-benzoxazin-7-yle | 198-200 | - |
| 6 | H, H, H, H | 0 | H, $CF_3$, H, H, H | 4-méthyl-3-oxo-2*H*-benzoxazin-6-yle | 178-180 | - |
| 7 | H, H, H, H | 0 | H, $CF_3$, H, H, H | 2-oxo-3,4-dihydroquinoléin-7-yle | $[MH]^+$ = 450 | - |
| 8 | H, H, H, H | 0 | H, $CF_3$, H, H, H | benzofuran-5-yle | 141-143 | - |
| 9 | H, H, H, H | 0 | H, CF3, H, H, H | 1-méthyl-indolin-5-yle | $[MH]^+$= 436 | - |
| 10 | H, H, H, H | 0 | H, $CF_3$, H, H, H | 2,3-dihydrobenzoxazin-6-yle | 90-92 | - |
| 11 | H, H, H, H | 0 | H, $CF_3$, H, H, H | 3-oxo-2*H*-benzoxazin-7-yle | 214-216 | - |
| 12 | H, F, H, H | 1 | H,F,H,H,H | 1-méthyl-indolin-5-yle | 145-147 | - |
| 13 | H,F,H,H | 1 | H, F, H, H, H | 1-méthyl-1,2,3,4-tétrahydroquinoléin-7-yle | 159-160 | - |
| 14 | H,F,H,H | 1 | H, F, H, H, H | 1,2,3,4-tétrahydroquinoléin-7-yle | 149-151 | - |
| 15 | H,F,H,H | 1 | H, F, H, H, H | isoquinoléin-5-yle | 206-207 | - |
| 16 | H, H, H, H | 0 | H, $CF_3$, H, H, H | 1,2,3,4-trétrahydroquinoléin-8-yle | 93-95 | - |
| 17 | H, H, H, H | 0 | H, $CF_3$, H, H, H | benzoxazol-5-yle | 222-224 | - |
| 18 | H, H, H, H | 0 | H, $CF_3$, H, H, H | 2-méthyl-benzoxazol-5-yle | 223-225 | - |
| 19 | H, $CF_3$, H, H | 1 | H, F, H, H, H | 1-méthyl-indazol-5-yle | 198-199 | - |
| 20 | H, F, H, H | 1 | H, F, H, H, H | 2-oxo-3,4-dihydroquinoléin-7-yle | 277 - 279 | - |

(suite)

| N° | $X_1, X_2, X_3, X_4$ | n | $Z_1, Z_2, Z_3, Z_4, Z_5$ | W | PF (°C) | Sel |
|---|---|---|---|---|---|---|
| 21 | H,F,H,H | 1 | H, F, H, H, H | benzofuran-5-yle | $[MH]^+$ = 403 | - |
| 22 | H, F, H, H | 1 | H, F, H, H, H | 2,3-dihydrobenzoxazin-6-yle | 216-217 | - |
| 23 | H, F, H, H | 1 | H, F, H, H, H | 3-oxo-2*H*-benzoxazin-6-yle | $[MH]^+$ = 434 | - |
| 24 | H, $CF_3$, H, H | 1 | H, F, H, H, H | 1,2,3,4-tétrahydroquinoléln-7-yle | 203-204 | - |
| 25 | H, $CF_3$, H, H | 1 | H,F,H,H,H | 2-oxo-indolin-5-yle | 244-246 | - |
| 26 | H, $CF_3$, H, H | 1 | H, F, H, H, H | 1-méthyl-benzimidazol-5-yle | 223-224 | - |
| 27 | H, $CF_3$, H, H | 1 | H, F, H, H, H | 1-méthyl-1,2,3,4-tétrahydroquinoléin-7-yle | 146-147 | - |
| 28 | H, $CF_3$, H, H | 1 | H, F, H, H, H | benzothiazol-6-yle | 191-192 | - |
| 29 | H, $CF_3$, H, H | 1 | H, F, H, H, H | 2-méthyl-benzoxazol-5-yle | 182-183 | - |
| 30 | H, $CF_3$, H, H | 1 | H, F, H, H, H | 2-méthyl-benzothiazol-5-yle | 191-192 | - |
| 31 | H, $CF_3$, H, H | 1 | H,F,H,H,H | 1-méthylsulfonyl-indolin-5-yle | 214-215 | - |
| 32 | H, F, H, H | 1 | H, F, H, H, H | isoquinoléin-6-yle | 139-141 | - |
| 33 | H, F, H, H | 1 | H, F, H, H, H | 1-méthyl-benzimidazol-5-yle | 248-251 | - |
| 34 | H, F, H, H | 1 | H, F, H, H, H | 1-méthyl-benzimidazol-4-yle | 195-197 | - |
| 35 | H, $CF_3$, H, H | 1 | H, F, H, H, H | 1*H*-benzotriazol-5-yle | 167 -174 | - |
| 36 | H, F, H, H | 1 | H,F,H,H,H | quinoléin-6-yle | 208-210 | - |
| 37 | H, F, H, H | 1 | H,F,H,H,H | 1-méthyl-indazol-5-yle | 210-211 | - |
| 38 | H, F, H, H | 1 | H, F, H, H, H | 2-méthyl-benzoxazol-5-yle | 188-189 | - |
| 39 | H, F, H, H | 1 | H, F, H, H, H | benzothiazol-6-yle | 167-168 | - |
| 40 | H,F,H,H | 1 | H, F, H, H, H | 2-méthyl-benzothiazol-5-yle | 201-202 | - |
| 41 | H, $CF_3$, H, H | 1 | H,F,H,H,H | 2-oxo-3,4-dihydroquinoléin-7-yle | 298-299 | - |
| 42 | H,F,H,H | 1 | H,F,H,H,H | 2-oxo-indolin-5-yle | 249-250 | - |
| 43 | H, F, H, H | 1 | H, F, H, H, H | 1*H*-benzotriazol-5-yle | 220-221 | - |

(suite)

| N° | $X_1$, $X_2$, $X_3$, $X_4$ | n | $Z_1$, $Z_2$, $Z_3$, $Z_4$, $Z_5$ | W | PF (°C) | Sel |
|---|---|---|---|---|---|---|
| 44 | H, F, H, H | 1 | H, F, H, H, H | 1-méthylsulfonyl-indolin-5-yle | 192-193 | - |
| 45 | H,F,H,H | 1 | H, F, H, H, H | 1,2-diméthyl-benzimidazol-5-yle | 281 - 282 | - |
| 46 | H, $CF_3$, H, H | 1 | H, E, H, H, H | 2-éthyl-benzoxazol-5-yle | 187-189 | - |
| 47 | H, $CF_3$, H, H | 1 | H, F, H, H, H | 2-phényl-benzoxazol-5-yle | 194-195 | - |
| 48 | H,F,H,H | 1 | H, F, H, H, H | quinoxalin-6-yle | 188-189 | - |
| 49 | H, F, H, H H, F, H, H | 1 | H, F, H, H, H H, F, H, H, H | quinoléin-7-yle | 222-223 | - |
| 50 | H, F, H, H | 1 | H,F,H,H,H | isoquinoléin-7-yle | 270-272 | HC (1:1) l |
| 51 | H, F, H, H | 1 | H, F, H, H, H | 2-méthylbenzimidazol-5-yle | 195-200 | HCl (1:1) |
| 52 | H, F, H, H | 1 | H, F, H, H, H | benzimidazol-5-yle | 275-280 | HCl (1:1) |
| 53 | H, H, $CH_3O$, H | 1 | H, H, F, H, H | 2-oxo-3,4-dihydroquinoléin-7-yle | 255-256 | - |
| 54 | H, F, H, H | 1 | H, F, H, H, H | 1-méthylbenzimidazol-6-yle | 215-216 | - |

Les composés de l'invention ont été soumis à des essais pharmacologiques *in vitro* et *in vivo* qui ont mis en évidence leur intérêt comme substances à activités thérapeutiques.

<u>Test d'inhibition du courant induit par la capsaïcine sur les DRG de rat</u>

- Culture primaire de cellules de ganglions de racine dorsale (DRG) de rat :

Les neurones du DRG expriment naturellement le récepteur TRPV1.

Les cultures primaires de DRG de rats nouveaux nés sont préparées à partir de ratons de 1 jour. Brièvement, après dissection, les ganglions sont trypsinés et les cellules dissociées mécaniquement par trituration ménagée. Les cellules sont re-suspendues dans un milieu de culture basal Eagle contenant 10 % de sérum de veau foetal, 25 mM KCl, 2 mM glutamine, 100 μg/ml gentamicine et 50 ng/ml de NGF, puis déposées sur des lamelles de verre recouvertes de laminine (0.25 x 106 cellules par lamelle) qui sont ensuite placées dans des boîtes 12 puits Corning. Les cellules sont incubées à 37°C en atmosphère humidifiée contenant 5% de $CO_2$ et 95% d'air. De la cytosine β-D-arabinoside (1 μM) est ajoutée 48 h après la mise en culture, pour prévenir le développement des cellules non neuronales. Les lamelles sont transférées dans les chambres expérimentales pour les études de patch-clamp après 7-10 jours de culture.

- Electrophysiologie

Les chambres de mesure (volume 800 μl) contenant la préparation cellulaire sont placées sur la platine d'un microscope inversé (Olympus IMT2) équipé d'optiques Hoffman (Modulation Contrast, New York) et observées au grossissement de 400X. Les chambres sont continuellement perfusées par gravité (2,5 ml/min) à l'aide d'un distributeur de solutions acceptant 8 entrées et dont la sortie unique, constituée par un tube de polyéthylène (ouverture 500μm) est

placée à moins de 3 mm de la cellule étudiée. La configuration "cellule entière" de la technique de patch-clamp à été utilisée. Les pipettes en verre borosilicaté (résistance 5-10 MOhms) sont approchées de la cellule grâce à un micromanipulateur piézoélectrique 3D ( Burleigh, PC1000) . Les courants globaux (potentiel de membrane fixé à -60 mV) sont enregistrés avec un amplificateur Axopatch 1D (Axon Instruments, Foster city, Californie), connecté à un PC piloté par les logiciels de Pclamp8 (Axon Instrument). Les traces de courant sont enregistrées sur papier et simultanément digitalisées (fréquence d'échantillonnage 15 à 25 Hz) et acquises sur le disque dur du PC.
L'application d'une solution de capsaïcine 300 nM, provoque sur les cellules de DRG (voltage fixé à -70 mV) un courant cationique entrant. Afin de minimiser la désensibilisation des récepteurs, l'intervalle d'une minute minimum entre deux applications de capsaïcine est respecté. Après une période contrôle (stabilisation de la réponse capsaïcine seule), les composés à tester sont appliqués seuls à une concentration donnée (concentration de 10 nM ou de 1 nM) pendant une durée de 4 à 5 minutes, au cours desquelles plusieurs tests capsaïcine + composé sont réalisés (obtention de l'inhibition maximale). Les résultats sont exprimés en % d'inhibition de la réponse capsaïcine contrôle.
Les pourcentages d'inhibition de la réponse capsaïcine (300 nM) sont compris entre 20% et 100% pour les composés antagonistes les plus actifs de l'invention testés à la concentration de 10 nM ou de 1 nM (voir quelques exemples dans le tableau 2).

**Tableau 2**

| N°composé | % inhibition en patch DRG |
|---|---|
| 14 | 38% (10nM) |
| 19 | 48% (1 nM) |
| 20 | 45% (1 nM) |

L'effet propre agoniste des composés peut être évalué par la mesure du courant induit à différentes concentrations de composé sur le DRG de rat, en présence ou non de capsazépine.

Test d'irritation cornéenne souris

Le caractère irritant de la capsaïcine est aisément apprécié au niveau de la cornée puisque cet organe est un des plus innervés par les fibres C. Dans ce contexte, d'après des expériences préliminaires, l'application d'une très faible quantité de capsaïcine (2 μl à une concentration de 160 μM) à la surface de la cornée d'un animal entraîne un certain nombre de comportements stéréotypés liés à l'irritation et qu'il est facile de répertorier. Parmi ceux-ci, on note : clignement de l'oeil, frottement de l'oeil instillé par la patte avant ipsilatérale, frottement de la face avec les deux pattes avant, grattement de la face ipsilatérale par la patte arrière. La durée de ces comportements ne dépasse pas les 2 minutes d'observation, et l'animal reprend alors son activité normale. Son aspect est par ailleurs également normal. La souris n'est pas recluse dans un coin avec les poils hérissés et ne développe aucun signe observable de souffrance. On peut en conclure que la durée d'action de la capsaïcine à ces doses est inférieure à 2 minutes.

Résumé de la méthodologie :

Le principe de la série d'expériences est de déterminer si les composés de l'invention peuvent influencer la réponse comportementale induite par une quantité donnée de capsaïcine. La capsaïcine est initialement diluée à 25 mM dans le DMSO et diluée, pour son utilisation finale, dans du Tween 80 à 10% dans le sérum physiologique. Il apparaît, à partir d'études contrôles que dans ces conditions, le solvant n'a aucun effet En pratique, le produit à tester est administré par voie orale, et, avec un délai (temps de prétraitement: t) qui déprend des données de pharmacocinétique, l'animal reçoit l'instillation oculaire de 2 μl d'une solution de capsaïcine à 160 μM préparée comme indiqué ci-dessus. Au cours d'une observation de 2 minutes suivant l'instillation, le nombre de frottements de l'oeil instillé par la patte antérieure ipsilatéral est répertorié.
Pour un animal donné, le pourcentage de protection est calculé comme suit':

**P= 100 – ((nombre de grattages observés / nombre moyen de grattages du groupe traité par le solvant) x 100)**

Ce pourcentage de protection est moyenné pour chaque groupe d'animaux (n = nombre d'animaux testés avec le

composé de l'invention).
Les pourcentages de protection évalués, dans ce modèle, pour les composés de l'invention les plus actifs, utilisés à la dose de 1 mg/kg (*po*), sont compris entre 20% et 100% (voir quelques exemples dans le tableau 3) :

**Tableau 3**

| n°composé | % P - (t) à 1 mg/kg (*po*) - (n=8) |
|---|---|
| 14 | 46%-(1h) |
| 20 | 26%-(1h) |

Les résultats de ces essais montrent que les composés peuvent avoir des effets agoniste ou antagoniste sur le récepteur TRPV1. Les composés antagonistes les plus actifs de l'invention bloquent les effets induits par la stimulation des récepteurs TRPV1. Les composés de l'invention peuvent donc être utilisés pour la préparation de médicaments, notamment pour la préparation d'un médicament destiné à prévenir ou à traiter les pathologies dans lesquelles les récepteurs de type TRPV1 sont impliqués.

Ainsi, selon un autre de ses aspects, l'invention a pour objet des médicaments qui comprennent un composé de formules (I), ou un sel pharmaceutiquement acceptable, ou encore un hydrate ou un solvat dudit composé.

Ces médicaments trouvent leur emploi en thérapeutique, notamment dans la prévention et/ou le traitement de la douleur et de l'inflammation, de la douleur chronique, neuropathique (traumatique, diabétique, métabolique, infectieuse, toxique, induite par un traitement anticancéreux ou hiatrogène), (ostéo-) arthritique, rhumatismale, des fibromyalgies, de la douleur, du dos, de la douleur liée au cancer, de la névralgie faciale, des céphalées, de la migraine, de la douleur dentaire, de la brûlure, du coup de soleil, de la morsure ou de la piqûre, de la nevralgie post-herpétique, de la douleur musculaire, de la compression nerveuse (centrale et/ou périphérique), des traumatismes de la moelle et/ou du cerveau, de l'ischémie (de la moelle et/ou du cerveau), de la neurodégénération, des accidents vasculaires hémorragiques (de la moelle et/ou du cerveau), de la douleur post-stroke.
Les composés de l'invention peuvent être utilisés pour la préparation d'un médicament destiné à prévenir et/ou à traiter les désordres urologiques tels que l'hyperactivité de la vessie, l'hyperéflexie vésicale, l'instabilité vésicale, l'incontinence, la miction d'urgence, l'incontinence urinaire, la cystite, la colique néphrétique, l'hypersensibilité pelvienne et la douleur pelvienne.
Les composés de l'invention peuvent être utilisés pour la préparation d'un médicament destiné à prévenir et/ou à traiter les désordres gynécologiques comme la vulvodynie, les douleurs liées aux salpingites, aux dysménorrhées.
On peut également utiliser ces produits pour la préparation d'un médicament destiné à prévenir et/ou à traiter les désordres gastrointestinaux tels que le désordre du réflexé gastroesophagique, l'ulcère de l'estomac, l'ulcère du duodénum, la dyspepsie fonctionnelle, la colite, l'IBS, la maladie de Crohn, la pancréatite, l'oesophagite, la colique hépatique.
De même, les produits de la présente invention peuvent être utiles dans la prévention et/ou le traitement des désordres respiratoires tels que l'asthme, la toux, la COPD, la bronchoconstriction et les désordres inflammatoires. Ces produits peuvent également être utilisés pour prévenir et/ou traiter le psoriasis, le pruritis, les irritations dermiques, des yeux ou des muqueuses, l'herpès, le zona.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable. Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies citées ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,001 à 30 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

1. Composé répondant à la formule (I)

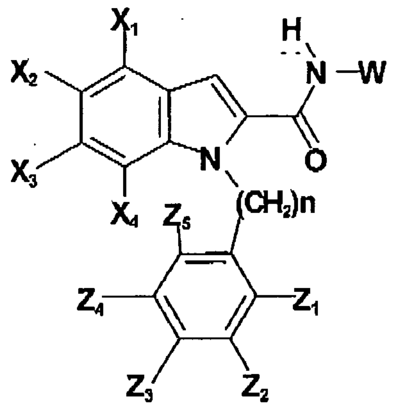

(I)

dans laquelle

n est égal à 0, 1, 2 ou 3 ;

$X_1$, $X_2$, $X_3$, $X_4$, $Z_1$, $Z_2$, $Z_3$, $Z_4$ et $Z_5$ représentent, indépendamment l'un de l'autre, un atome d'hydrogène ou d'halogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxy, $C_1$-$C_6$-fluoroalcoxy, cyano, $C(O)NR_1R_2$, nitro, $NR_1R_2$, $C_1$-$C_6$-thioalkyle, -S(O)-$C_1$-$C_6$-alkyle, -$S(O)_2$-$C_1$-$C_6$-alkyle, $SO_2NR_1R_2$, $NR_3COR_4$, $NR_3SO_2R_5$ ou aryle, l'aryle étant éventuellement substitué par un ou plusieurs substitutants choisi parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxy, $C_1$-$C_6$-fluoroalcoxy, nitro ou cyano ;

$R_1$ et $R_2$, représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_3$-alkylène ou aryle ; ou $R_1$ et $R_2$ formant ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe $C_1$-$C_6$-alkyle, $C_3$-$C_7$-cycloalkyle, $C_3$-$C_7$-cycloalkyle-$C_1$-$C_3$-alkylène ou aryle ;

$R_3$ et $R_4$ représentent, indépendamment l'un de l'autre, un atome d'hydrogène ou un groupe $C_1$-$C_6$-alkyle ou aryle ;

$R_5$ représente un groupe $C_1$-$C_6$-alkyle ou aryle ;

W représente un groupe bicyclique fusionné de formule :

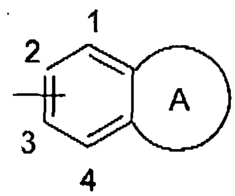

lié à l'atome d'azote par les positions 1, 2, 3 ou 4 ;
A représente un hétérocycle de 5 à 7 chaînons comprenant de un à trois hétéroatomes choisi parmi O, S ou N ;
le ou les atomes de carbones de A étant éventuellement substitués par un ou plusieurs groupes choisis parmi un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle, aryle-$C_1$-$C_6$-alkylène, oxo ou thio;
le ou les atomes d'azote de A étant éventuellement substitués par $R_6$ lorsque l'azote est adjacent à un atome de carbone substitué par un groupe oxo, ou par $R_7$ dans les autres cas ;
$R_6$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène ou aryle ;
$R_7$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkyléne, $C_1$-$C_6$-fluoroalkyle, aryle-$C_1$-$C_6$-alkylène, $C_1$-$C_6$-alkyle-C(O)-, $C_3$-$C_7$-cycloalkyle-$C_1$-$C_3$-alkylène-(CO)-, $C_1$-$C_6$-fluoroalkyle-C(O)-, $C_3$-$C_7$-cycloalkyle-C(O)-, aryle-C(O)-, aryle-$C_1$-$C_6$-alkylène-C(O)-, $C_1$-$C_6$-alkyle-S$(O)_2$-, $C_1$-$C_6$-fluoroalkyle-S$(O)_2$-, $C_3$-$C_7$-cycloalkyle-S$(O)_2$-, $C_3$-$C_7$-cycloalkyle-$C_1$-$C_3$-alkylène-S$(O)_2$-, aryle-S$(O)_2$- ou aryle-$C_1$-$C_6$-alkylène-S$(O)_2$- ou aryle ; et
W est différent de l'indolyle ;
le ou les atomes de soufre de l'hétérocycle A pouvant être sous forme oxydée ;
le ou les atomes d'azote de l'hétérocycle A pouvant être sous forme oxydée ;
à l'état de base ou de sel d'addition à un acide, ainsi qu'à l'état d'hydrate ou de solvat.

**2.** Composé de formule (I) selon la revendication 1, **caractérisé en ce que** n est égal à 0 ou 1, à l'état de base ou de sel d'addition à un acide, ainsi qu'à l'état d'hydrate ou de solvat.

**3.** Composé de formule (I) selon la revendication 1 ou 2, **caractérisé en ce que** $X_1$, $X_2$, $X_3$, $X_4$, $Z_1$, $Z_2$, $Z_3$, $Z_4$ et $Z_5$ représentent, indépendamment l'un de l'autre, un atome d'hydrogène ou d'halogène ou un groupe $C_1$-$C_6$-alkyle, $C_1$-$C_6$-fluoroalkyle ou $C_1$-$C_6$-alcoxy, à l'état de base ou de sel d'addition à un acide, ainsi qu'à l'état d'hydrate ou de solvat.

**4.** Composé de formule (I) selon l'une quelconque des revendications 1 à 3, **caractérisé en ce que** W est choisi parmi les groupes indolinyle, isoindolinyle, benzofuranyle, dihydrobenzofuranyle, benzothiophényle, dihydrobenzothiophényle, benzoxazolyle, dihydrobenzoxazolinyle, isobenzofuranyle, dihydroisobenzofuranyle, benzimidazolyle, dihydrobenzimidazolyle, indazolyle, benzothiazolyle, isobenzothiazolyle, dihydroisobonzothiazolyle, benzotriazolyle, quinoléinyle, dihydroquinoléinyle, tétrahydroquinoléinyle, isoquinoléinyle, dihydroisoquinoléinyle, tétrahydroisoquinoléinyle, benzoxazinyle, dihydrobenzoxazinyle, benzothiazinyle, dihydrobenzothiazinyle, cinnolinyle, quinazolinyle, dihydroquinazolinyle, tétrahydroquinazolinyle, quinoxalinyle, dihydroquinoxalinyle, tétrahydroquinoxalinyle, phtalazinyle, dihydrophtalazinyle, tétrahydrophtalazinyle, tétrahydrobenz[*b*]azépinyle, tétrahydrobenz[*c*]azépinyle, tétrahydrobenz[*d*]azépinyle, tétrahydrobenzo[*b*][1,4]diazépinyle, tétrahydrobenzo[*e*][1,4]diazépinyle, tétrahydrobenzo[*b*][1,4]oxazépinyle ou tétrahydrobenzo[*b*][1,4]thiazépinyle ; le ou les atomes de carbone et/ou d'azote dudit groupe W étant éventuellement substitués comme défini dans la formule générale (I) selon la revendication 1 ; à l'état de base ou de sel d'addition à un acide, ainsi qu'à l'état d'hydrate ou de solvat.

**5.** Composé de formule (I) selon la revendication 4, **caractérisé en ce que** W est choisi parmi les groupes isoquinoléinyle, dihydroquinoléinyle, tétrahydroquinoléinyle, benzoxazinyle, dihydrobenzoxazinyle, benzofuranyle, indolinyle, benzoxazolyle, indazolyle, benzimidazolyle, benzothiazolyle, benzotriazolyle, quinoléinyle, quinoxalinyle ; le ou les atomes de carbone dudit groupe W étant éventuellement substitués par un ou plusieurs groupes choisis parmi un groupe oxo, $C_1$-$C_6$-alkyle ou aryle, tels que définis dans la formule générale (I) en relation avec A ; et/ou

le ou les atomes d'azote dudit groupe W étant éventuellement substitués par $R_6$ lorsque l'azote est adjacent à un atome de carbone substitué par un groupe oxo, ou par $R_7$ dans les autres cas, $R_6$ et $R_7$ étant tels que définis dans la formule générale (I) selon la revendication 1 en relation avec A,
avec $R_6$ représentant un atome d'hydrogène ou un groupe $C_1$-$C_6$-alkyle,

avec $R_7$ représentant un atome d'hydrogène ou un groupe $C_1$-$C_6$-alkyle ou un $C_1$-$C_6$-alkyle-$S(O)_2$- ; à l'état de base ou de sel d'addition à un acide, ainsi qu'à l'état d'hydrate ou de solvat.

**6.** Composé de formule (I) selon la revendication 1 choisi parmi :

*N*-(isoquinoléin-5-yl)-5-fluoro-1-[((3-trifluorométhyl)phényl)méthyl]-1*H*-indole-2-carboxamide,
*N*-(1-méthyl-1,2,3,4-tétrahydroquinoléin-7-yl)-1-[3-(trifluorométhyl)phényl]-1*H*-indole-2-carboxamide,
*N*-(1-méthyl-1,2,3,4-tétrahydroquinoléin-7-yl)-1-((3-5-diméthyl)phényl)-1*H*-indole-2-carboxamide,
*N*-(1,2,3,4-tétrahydroquinoléin-7-yl)-1-[3-(trifluorométhyl)phényl]-1*H*-indole-2-carboxamide,
*N*-(4-méthyl-3-oxo-2*H*-benzoxazin-7-yl)-1-[3,(trifluorométhyl)phényl]-1*H*-indole-2-carboxamide,
*N*-(4-méthyl-3-oxo-2*H*-benzoxazin-6-yl)-1-[3-(trifluorométhyl)phényl]-1*H*-indole-2-carboxamide,
*N*-(2-oxo-3,4-dihydroquinoléin-7-yl)-1-[3-(trifluorométhyl)phényl]-1*H*-indole-2-carboxamide,
*N*-(benzofuran-5-yl)-1-[3-(trifluorométhyl)phényl]-1*H*-indole-2-carboxamide,*N*-(1-méthyl-indolin-5-yle)-1-[3-(trifluorométhyl)phényl]-1*H*-indole-2-carboxamide,
*N*-(2,3-dihydrobenzoxazin-6-yl)-1-[3-(trifluorométhyl)phényl]-1*H*-indole-2-carboxamide,
*N*-(3-oxo-2*H*-benzoxazin-7-yl)-1-[3-(trifluorométhyl)phényl]-1*H*-indole-2-carboxamide,
*N*-(1-méthyl-indolin-5-yl)-5-fluoro-1-[(3-fluorophényl)méthyl]-1*H*-indole-2-carboxamide,
*N*-(1-méthyl-1,2,3,4-tétrahydroquinoléin-7-yl)-5-fluoro-1-[(3-fluorophényl)méthyl]-1*H*-indole-2-carboxamide,
*N*-(1,2,3,4-tétrahydroquinoléin-7-yl)-5-fluoro-1-[(3-fluorophényl)méthyl]-1*H*-indole-2-carboxamide,
*N*-(isoquinoléin-5-yl)-5-fluoro-1-[(3-fluorophényl)méthyl]-1*H*-indole-2-carboxamide,
*N*-(1,2,3,4-tétrahydroquinoléin-8-yl)-1-[3-(trifluorométhyl)phényl]-1*H*-indole-2-carboxamide,
*N*-(benzoxazol-5-yl)-1-[3-(trifluorométhyl)phényl]-1*H*-indole-2-carboxamide,
*N*-(2-méthyl-benzoxazol-5-yl)-1-[3-(trifluorométhyl)phényl]-1*H*-indole-2-carboxamide,
*N*-(1-méthyl-1*H*-indazol-5-yl)-5-trifluorométhyl-1-[(3-fluorophényl)méthyl]-1*H*-indole-2-carboxamide,
*N*-(2-oxo-3,4-dihydroquinoléin-7-yl)-5-fluoro-1-[(3-fluorophényl)méthyl]-1*H*-indole-2-carboxamide,
*N*-(benzofuran-5-yl)-5-fluoro-1-[(3-fluorophényl)méthyl]-1*H*-indole-2-carboxamide,
*N*-(2,3-dihydrobenzoxazin-6-yl)-5-fluoro-1-[(3-fluorophényl)méthyl]-1*H*-indole-2-carboxamide,
*N*-(3-oxo-2*H*-benzoxazin-6-yl)-5-fluoro-1-[(3-fluorophényl)méthyl]-1*H*-indole-2-carboxamide,
*N*-(1,2,3,4-tétrahydroquinoléin-7-yl)-5-trifluorométhyl-1-[(3-fluorophényl)méthyl]-1*H*-indole-2-carboxamide,
*N*-(2-oxo-indolin-5-yl)-5-trifluorométhyl-1-[(3-fluorophényl)méthyl]-1*H*-indole-2-carboxamide,
*N*-(1-méthyl-benzimidazol-6-yl)-5-trifluorométhyl-1-[(3-fluorophényl)méthyl]-1*H*-indole-2-carboxamide,
*N*-(1-méthyl-1,2,3,4-tétrahydroquinoléin-7-yl)-5-trifluorométhyl-1-[(3-fluorophényl)méthyl]-1*H*-indole-2-carboxamide,
*N*-(benzothiazol-6-yl)-5-trifluorométhyl-1-[(3-fluorophényl)méthyl]-1*H*-indole-2-carboxamide,
*N*-(2-méthyl-benzoxazol-5-yl)-5-trifluorométhyl-1-[(3-fluorophényl)méthyl]-1*H*-indole-2-carboxamide,
*N*-(2-méthyl-benzothiazol-5-yl)-5-trifluorométhyl-1-[(3-fluorophényl)méthyl]-1*H*-indole-2-carboxamide,
*N*-(1-méthylsulfonyl-indolin-5-yl)-5-trifluorométhyl-1-[(3-fluorophényl)méthyl]-1*H*-indole-2-carboxamide,
*N*-(isoquinoléin-6-yl)-5-fluoro-1-[(3-fluorophényl)méthyl]-1*H*-indole-2-carboxamide,
*N*-(1-méthyl-benzimidazol-5-yl)-5-fluoro-1-[(3-fluorophényl)méthyl]-1*H*-indole-2-carboxamide,
*N*-(1-méthyl-benzimidazol-4-yl)-5-fluoro-1-[(3-fluorophényl)méthyl]-1*H*-indole-2-carboxamide,
*N*-(1*H*-benzotriazol-5-yl)-5-trifluorométhyl-1-[(3-fluorophényl)méthyl]-1*H*-indole-2-carboxamide,
*N*-(quinoléin-6-yl)-5-fluoro-1-[(3-fluorophényl)méthyl]-1*H*-indole-2-carboxamide,
*N*-(1-méthyl-indazol-5-yl)-5-fluoro-1-[(3-fluarophényl)méthyl]-1*H*-indole-2-carboxamide,
*N*-(2-méthyl-benzoxazol-5-yl)-5-fluoro-1-[(3-fluorophényl)méthyl]-1*H*-indole-2-carboxamide,
*N*-(benzothiazol-6-yl)-5-fluoro-1-[(3-fluorophényl)méthyl]-1*H*-indole-2-carboxamide,
*N*-(2-méthyl-benzothiazol-5-yl)-5-fluoro-1-[(3-fluorophényl)méthyl]-1*H*-indole-2-carboxamide,
*N*-(2-oxo-3,4-dihydroquinoléin-7-yl)-5-trifluorométhyl-1-[(3-fluorophényl)méthyl]-1*H*-indole-2-carboxamide,
*N*-(2-oxo-indolin-5-yl)-5-fluoro-1-[(3-fluorophényl)méthyl]-1*H*-indole-2-carboxamide,
*N*-(1*H*-benzotriazol-5-yl)-5-fluoro-1-[(3-fluorophényl)méthyl]-1*H*-indole-2-carboxamide,
*N*-(1-méthylsulfonyl-indolin-5-yl)-5-fluoro-1-[(3-fluorophényl)méthyl]-1*H*-indole-2-carboxamide,
*N*-(1,2-diméthyl-benzimidazol-5-yl)-5-fluoro-1-[(3-fluorophényl)méthyl]-1*H*-indole-2-carboxamide,
*N*-(2-éthyl-benzoxazol-5-yl)-5-trifluorométhyl-1-[(3-fluorophényl)méthyl]-1*H*-indole-2-carboxamide,
*N*-(2-phényl-benzoxazol-5-yl)-5-trifluorométhyl-1-[(3-fluorophényl)méthyl]-1*H*-indole-2-carboxamide,
*N*-(quinoxalin-6-yl)-5-fluoro-1-[(3-fluorophényl)méthyl]-1*H*-indole-2-carboxamide,
*N*-(quinoléin-7-yl)-5-fluoro-1-[(3-fluorophényl)méthyl]-1*H*-indole-2-carboxamide,
*N*-(isoquinoléin-7-yl)-5-fluoro-1-[(3-fluorophényl)méthyl]-1*H*-indole-2-carboxamide,
*N*-(2-méthylbenzimidazol-5-yl)-5-fluoro-1-[(3-fluorophényl)méthyl]-1*H*-indole-2-carboxamide,
*N*-(benzimidazol-5-yl)-5-fluoro-1-[(3-fluorophényl)méthyl]-1*H*-indole-2-carboxamide,

*N*-(2-oxo-3,4-dihydroquinoléin-7-yl)-6-méthoxy-1-[(4-fluorophényl)méthyl]-1*H*-indole-2-carboxamide et *N*-(1-méthylbenzimidazol-6-yl)-5-fluoro-1-[(3-fluorophényl)méthyl]-1*H*-indole-2-carboxamide.

**7.** Procédé de préparation d'un composé de formule (I) selon l'une quelconque des revendications 1 à 6, **caractérisé en ce que** l'on fait réagir un composé de formule générale (IV)

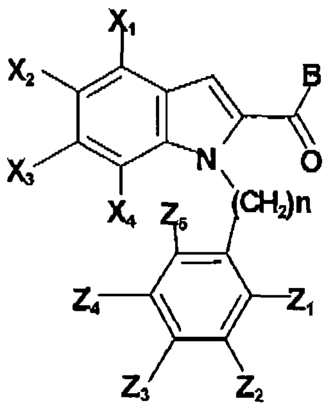

**(IV)**

dans laquelle $X_1$, $X_2$, $X_3$, $X_4$, $Z_1$, $Z_2$, $Z_3$, $Z_4$, $Z_5$ et n sont tels que définis dans la formule générale (I) selon la revendication 1 et B représente un groupe $C_1$-$C_4$-alcoxy,
avec un amidure du composé de formule générale (V)

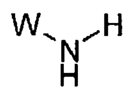

dans laquelle W est tel que défini dans la formule générale (I) selon la revendication 1, au reflux d'un solvant, l'amidure du composé de formule générale (V) étant préparé par action préalable du triméthylaluminium sur les composés de formule générale (V).

**8.** Procédé de préparation d'un composé de formule (I) selon l'une quelconque des revendications 1 à 6, **caractérisé en ce que** l'on transforme un composé de formule générale (IV)

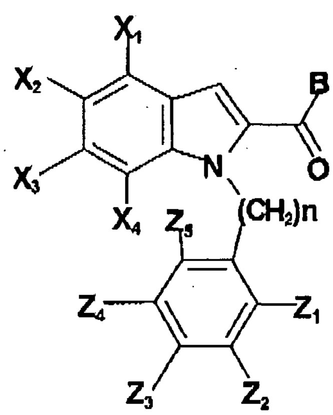

**(IV)**

dans laquelle $X_1$, $X_2$, $X_3$. $X_4$, $Z_1$, $Z_2$, $Z_3$, $Z_4$, $Z_5$ et n sont tels que définis dans la formule générale (I) selon la revendication 1 et B représente un groupe hydroxy,
en chlorure d'acide par action du chlorure de thionyle au reflux d'un solvant,
puis **en ce que** l'on fait réagir, en présence d'une base, le composé de formule générale (IV) obtenu, dans laquelle $X_1$, $X_2$, $X_3$, $X_4$, $Z_1$, $Z_2$, $Z_3$, $Z_4$, $Z_5$ et n sont tels que définis dans la formule générale (I) selon la revendication 1 et B représente un atome de chlore, avec le composé de formule générale (V),

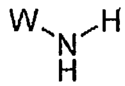

dans laquelle W est tel que défini dans la formule générale (I) selon la revendication 1, ou bien **en ce que** l'on effectue une réaction de couplage entre un composé de formule générale (IV) dans laquelle $X_1$, $X_2$, $X_3$, $X_4$, $Z_1$, $Z_2$, $Z_3$, $Z_4$, $Z_6$ et n sont tels que définis dans la formule générale (I) selon la revendication 1 et B représente un groupe hydroxy,
et le composé de formule générale (V), dans laquelle W est tel que défini dans la formule générale (I) selon la revendication 1, en présence d'un agent de couplage et d'une base, dans un solvant.

**9.** Médicament, **caractérisé en ce qu'**il comprend un composé de formule (1), selon l'une quelconque des revendications 1 à 6, ou un sel pharmaceutiquement acceptable, ou encore un hydrate ou un solvat du composé de formule (I).

**10.** Composition pharmaceutique, **caractérisée en ce qu'**elle comprend un composé de formule (I), selon l'une quelconque des revendications 1 à 6, ou un sel pharmaceutiquement acceptable, un hydrate ou un solvat de ce composé, ainsi qu'au moins un excipient pharmaceutiquement acceptable.

**11.** Utilisation d'un composé de formule (I), selon l'une quelconque des revendications 1 à 6, pour la préparation d'un médicament destiné à prévenir ou à traiter les pathologies dans lesquelles les récepteurs de type TRPV1 sont impliqués.

**12.** Utilisation d'un composé de formule (I), selon l'une quelconque des revendications 1 à 6, pour la préparation d'un médicament destiné à prévenir ou à traiter la douleur et l'inflammation, les désordres urologiques, les désordres gynécologiques, les désordres gastrointestinaux, des désordres respiratoires, le psoriasis, le pruritis, les irritations dermiques, des yeux ou des muqueuses, l'herpès, le zona.

**13.** Utilisation d'un composé de formule (I), selon la revendication 12, **caractérisé en ce que** la douleur et l'inflammation sont de nature chronique, neuropathique (traumatique, diabétique, métabolique, infectieuse, toxique, induite par un traitement anticancéreux ou hiatrogène), (ostéo-) arthritique, rhumatismale, ou bien de type fibromyalgies, douleur du dos, douleur liée au cancer, névralgie faciale, céphalées, migraine, douleur dentaire, brûlure, coup de soleil, morsure ou piqûre, nevralgie post-herpétique, douleur musculaire, compression nerveuse (centrale et/ou périphérique), traumatismes de la moelle et/ou du cerveau, ischémie (de la moelle et/ou du cerveau), neurodégénération, accidents vasculaires hémorragiques (de la moelle et/ou du cerveau), douleur post-stroke.

**14.** Utilisation d'un composé de formule (I), selon la revendication 12, **caractérisé en ce que** les désordres urologiques sont choisis parmi l'hyperactivité de la vessie, l'hyperéflexie vésicale, l'instabilité vésicale, l'incontinence, la miction d'urgence, l'incontinence urinaire, la cystite, la colique néphrétique, l'hypersensibilité pelvienne et la douleur pelvienne.

**15.** Utilisation d'un composé de formule (I), selon la revendication 12, **caractérisé en ce que** les désordres gynécologiques sont choisis parmi la vulvodynie, les douleurs liées aux salpingites, aux dysménorrhées.

**16.** Utilisation d'un composé de formule (**I**), selon la revendication 12, **caractérisé en ce que** les désordres gastrointestinaux sont choisis parmi le désordre du réflexe gastroesophagique, l'ulcère de l'estomac, l'ulcère du duodénum, la dyspepsie fonctionnelle, la colite, l'IBS, la maladie de Crohn, la pancréatite, l'oesophagite, la colique hépatique.

**17.** Utilisation d'un composé de formule (I), selon la revendication 12, **caractérisé en ce que** les désordres respiratoires sont choisis parmi l'asthme, la toux, la COPD, la bronchoconstriction et les désordres inflammatoires.

## Claims

**1.** Compound corresponding to formula (I)

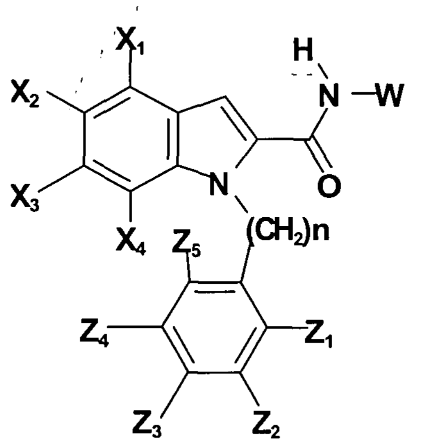

(I)

in which

n is equal to 0, 1, 2 or 3;
$X_1$, $X_2$, $X_3$, $X_4$, $Z_1$, $Z_2$, $Z_3$, $Z_4$ and $Z_5$ represent, independently of each other, a hydrogen or halogen atom or a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, $C_1$-$C_6$-alkoxy, $C_1$-$C_6$-fluoroalkoxy, cyano, C(O)$NR_1R_2$, nitro, $NR_1R_2$, $C_1$-$C_6$-thioalkyl, -S (O) -$C_1$-$C_6$-alkyl, -S $(O)_2$-$C_1$-$C_6$-alkyl, $SO_2NR_1R_2$, $NR_3COR_4$, $NR_3SO_2R_5$ or aryl group, the aryl being optionally substituted with one or more substituents chosen from a halogen and a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, $C_1$-$C_6$-alkoxy, $C_1$-$C_6$-fluoroalkoxy, nitro or cyano group;
$R_1$ and $R_2$ represent, independently of each other, a hydrogen atom or a $C_1$-$C_6$-alkyl, $C_3$-$C_7$-cycloalkyl, $C_3$-$C_7$-cycloalkyl-$C_1$-$C_3$-alkylene or aryl group; or $R_1$ and $R_2$ form, together with the nitrogen atom that bears them, an azetidine, pyrrolidine, piperidine, azepine, morpholine, thiomorpholine, piperazine or homopiperazine group, this group being optionally substituted with a $C_1$-$C_6$-alkyl, $C_3$-$C_7$-cycloalkyl, $C_3$-$C_7$-cycloalkyl-$C_1$-$C_3$-alkylene or aryl group;
$R_3$ and $R_4$ represent, independently of each other, a hydrogen atom or a $C_1$-$C_6$-alkyl or aryl group;
$R_5$ represents a $C_1$-$C_6$-alkyl or aryl group;
W represents a fused bicyclic group of formula:

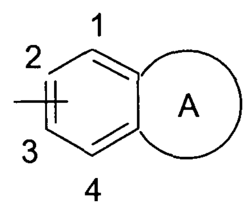

linked to the nitrogen atom via positions 1, 2, 3 or 4; A represents a 5- to 7-membered heterocycle comprising from one to three heteroatoms chosen from O, S and N; the carbon atom(s) of A being optionally substituted with one or more groups chosen from a hydrogen atom and a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, aryl, aryl-$C_1$-$C_6$-alkylene, oxo or thio group;
the nitrogen atom(s) of A being optionally substituted with $R_6$ when the nitrogen is adjacent to a carbon atom substituted with an oxo group, or with $R_7$ in the other cases;
$R_6$ represents a hydrogen atom or a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, aryl-$C_1$-$C_6$-alkylene or aryl group;
$R_7$ represents a hydrogen atom or a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, aryl-$C_1$-$C_6$-alkylene, $C_1$-$C_6$-alkyl-C(O)-, $C_3$-$C_7$-cycloalkyl-$C_1$-$C_3$-alkylene-(CO)-, $C_1$-$C_6$-fluoroalkyl-C(O)-, $C_3$-$C_7$-cycloalkyl-C(O) -, aryl-C(O)-, aryl-$C_1$-$C_6$-alkylene-C(O)-, $C_1$-$C_6$-alkyl-$S(O)_2$-, $C_1$-$C_6$-fluoroalkyl-$S(O)_2$-, $C_3$-$C_7$-cycloalkyl-$S(O)_2$-, $C_3$-$C_7$-cycloalkyl-$C_1$-$C_3$-alkylene-$S(O)_2$-, aryl-$S(O)_2$- or aryl-$C_1$-$C_6$-alkylene-$S(O)_2$- or aryl group; and
W is other than indolyl;
the sulfur atom(s) of the heterocycle A possibly being in oxidized form;

the nitrogen atom(s) of the heterocycle A possibly being in oxidized form;
in the form of base or of acid-addition salt, and also in the form of hydrate or solvate.

2. Compound of formula (I) according to Claim 1, **characterized in that** n is equal to 0 or 1, in the form of base or of acid-addition salt, and also in the form of hydrate or solvate.

3. Compound of formula (I) according to Claim 1 or 2, **characterized in that** $X_1$, $X_2$, $X_3$, $X_4$, $Z_1$, $Z_2$, $Z_3$, $Z_4$ and $Z_5$ represent, independently of each other, a hydrogen or halogen atom or a $C_1$-$C_6$-alkyl, $C_1$-$C_6$-fluoroalkyl or $C_1$-$C_6$-alkoxy group, in the form of base or of acid-addition salt, and also in the form of hydrate or solvate.

4. Compound of formula (I) according to any one of Claims 1 to 3, **characterized in that** W is chosen from indolinyl, isoindolinyl, benzofuryl, dihydrobenzofuryl, benzothiophenyl, dihydrobenzothiophenyl, benzoxazolyl, dihydrobenzoxazolinyl, isobenzofuryl, dihydroisobenzofuryl, benzimidazolyl, dihydrobenzimidazolyl, indazolyl, benzothiazolyl, isobenzothiazolyl, dihydroisobenzothiazolyl, benzotriazolyl, quinolyl, dihydroquinolyl, tetrahydroquinolyl, isoquinolyl, dihydroisoquinolyl, tetrahydroisoquinolyl, benzoxazinyl, dihydrobenzoxazinyl, benzothiazinyl, dihydrobenzothiazinyl, cinnolinyl, quinazolinyl, dihydroquinazolinyl, tetrahydroquinazolinyl, quinoxalinyl, dihydroquinoxalinyl, tetrahydroquinoxalinyl, phthalazinyl, dihydrophthalazinyl, tetrahydrophthalazinyl, tetrahydrobenz[b]azepinyl, tetrahydrobenz[c]azepinyl, tetrahydrobenz[d]azepinyl, tetrahydrobenzo[b][1,4]diazepinyl, tetrahydrobenzo-[e][1,4]diazepinyl, tetrahydrobenzo[b][1,4]oxazepinyl and tetrahydrobenzo[b][1,4]thiazepinyl groups;
the carbon and/or nitrogen atom(s) of the said group W optionally being substituted as defined in the general formula (I) according to Claim 1;
in the form of base or of acid-addition salt, and also in the form of hydrate or solvate.

5. Compound of formula (I) according to Claim 4, **characterized in that** W is chosen from isoquinolyl, dihydroquinolyl, tetrahydroquinolyl, benzoxazinyl, dihydrobenzoxazinyl, benzofuryl, indolinyl, benzoxazolyl, indazolyl, benzimidazolyl, benzothiazolyl, benzotriazolyl, quinolyl, quinoxalinyl groups;
the carbon atom(s) of the said group W being optionally substituted with one or more groups chosen from an oxo, $C_1$-$C_6$-alkyl or aryl group, as defined in the general formula (I) in relation with A; and/or
the nitrogen atom(s) of the said group W being optionally substituted with $R_6$ when the nitrogen is adjacent to a carbon atom substituted with an oxo group, or with $R_7$ in the other cases, $R_6$ and $R_7$ being as defined in the general formula (I) according to Claim 1 in relation with A,
with $R_6$ representing a hydrogen atom or a $C_1$-$C_6$-alkyl group,
with $R_7$ representing a hydrogen atom or a $C_1$-$C_6$-alkyl or $C_1$-$C_6$-alkyl-$S(O)_2$ group;
in the form of base or of acid-addition salt, and also in the form of hydrate or solvate.

6. Compounds of formula (I) according to Claim 1, chosen from formula (I):

N-(isoquinol-5-yl)-5-fluoro-1-[((3-trifluoromethyl)phenyl)methyl]-1H-indole-2-carboxamide,
N-(1-methyl-1,2,3,4-tetrahydroquinol-7-yl)-1-[3-(trifluoromethyl)phenyl]-1H-indole-2-carboxamide,
N-(1-methyl-1,2,3,4-tetrahydroquinol-7-yl)-1-((3-5-dimethyl)phenyl)-1H-indole-2-carboxamide,
N-(1,2,3,4-tetrahydroquinol-7-yl)-1-[3-(trifluoromethyl)phenyl]-1H-indole-2-carboxamide,
N-(4-methyl-3-oxo-2H-benzoxazin-7-yl)-1-[3-(trifluoromethyl)phenyl]-1H-indole-2-carboxamide,
N-(4-methyl-3-oxo-2H-benzoxazin-6-yl)-1-[3-(trifluoromethyl)phenyl]-1H-indole-2-carboxamide,
N-(2-oxo-3,4-dihydroquinol-7-yl)-1-[3-(trifluoromethyl)phenyl]-1H-indole-2-carboxamide,
N-(benzofuran-5-yl)-1-[3-(trifluoromethyl)phenyl]-1H-indole-2-carboxamide,
N-(1-methylindolin-5-yl)-1-[3-(trifluoromethyl)phenyl]-1H-indole-2-carboxamide,
N-(2,3-dihydrobenzoxazin-6-yl)-1-[3-(trifluoromethyl)phenyl]-1H-indole-2-carboxamide,
N-(3-oxo-2H-benzoxazin-7-yl)-1-[3-(trifluoromethyl)phenyl]-1H-indole-2-carboxamide,
N-(1-methylindolin-5-yl)-5-fluoro-1-[(3-fluorophenyl)methyl]-1H-indole-2-carboxamide,
N-(1-methyl-1,2,3,4-tetrahydroquinol-7-yl)-5-fluoro-1-[(3-fluorophenyl)methyl]-1H-indole-2-carboxamide,
N-(1,2,3,4-tetrahydroquinol-7-yl)-5-fluoro-1-[(3-fluorophenyl)methyl]-1H-indole-2-carboxamide,
N-(isoquinol-5-yl)-5-fluoro-1-[(3-fluorophenyl)methyl]-1H-indole-2-carboxamide,
N-(1,2,3,4-tetrahydroquinol-8-yl)-1-[3-(trifluoromethyl)phenyl]-1H-indole-2-carboxamide,
N-(benzoxazol-5-yl)-1-[3-(trifluoromethyl)phenyl]-1H-indole-2-carboxamide,
N-(2-methylbenzoxazol-5-yl)-1-[3-(trifluoromethyl)phenyl]-1H-indole-2-carboxamide,
N-(1-methyl-1H-indazol-5-yl)-5-trifluoromethyl-1-[(3-fluorophenyl)methyl]-1H-indole-2-carboxamide,
N-(2-oxo-3,4-dihydroquinol-7-yl)-5-fluoro-1-[(3-fluorophenyl)methyl]-1H-indole-2-carboxamide,
N-(benzofuran-5-yl)-5-fluoro-1-[(3-fluorophenyl)methyl]-1H-indole-2-carboxamide,

N-(2,3-dihydrobenzoxazin-6-yl)-5-fluoro-1-[(3-fluorophenyl)methyl]-1H-indole-2-carboxamide,
N-(3-oxo-2H-benzoxazin-6-yl)-5-fluoro-1-[(3-fluorophenyl)methyl]-1H-indole-2-carboxamide,
N-(1,2,3,4-tetrahydroquinol-7-yl)-5-trifluoromethyl-1-[(3-fluorophenyl)methyl]-1H-indole-2-carboxamide,
N-(2-oxoindolin-5-yl)-5-trifluoromethyl-1-[(3-fluorophenyl)methyl]-1H-indole-2-carboxamide,
N-(1-methylbenzimidazol-5-yl)-5-trifluoromethyl-1-[(3-fluorophenyl)methyl]-1H-indole-2-carboxamide,
N-(1-methyl-1,2,3,4-tetrahydroquinol-7-yl)-5-trifluoromethyl-1-[(3-fluorophenyl)methyl]-1H-indole-2-carboxamide,
N-(benzothiazol-6-yl)-5-trifluoromethyl-1-[(3-fluorophenyl)methyl]-1H-indole-2-carboxamide,
N-(2-methylbenzoxazol-5-yl)-5-trifluoromethyl-1-[(3-fluorophenyl)methyl]-1H-indole-2-carboxamide,
N-(2-methylbenzothiazol-5-yl)-5-trifluoromethyl-1-[(3-fluorophenyl)methyl]-1H-indole-2-carboxamide,
N-(1-methylsulfonylindolin-5-yl)-5-trifluoromethyl-1-[(3-fluorophenyl)methyl]-1H-indole-2-carboxamide,
N-(isoquinol-6-yl)-5-fluoro-1-[(3-fluorophenyl)methyl]-1H-indole-2-carboxamide,
N-(1-methylbenzimidazol-5-yl)-5-fluoro-1-[(3-fluorophenyl)methyl]-1H-indole-2-carboxamide,
N-(1-methylbenzimidazol-4-yl)-5-fluoro-1-[(3-fluorophenyl)methyl]-1H-indole-2-carboxamide,
N-(1H-benzotriazol-5-yl)-5-trifluoromethyl-1-[(3-fluorophenyl)methyl]-1H-indole-2-carboxamide,
N-(quinol-6-yl)-5-fluoro-1-[(3-fluorophenyl)methyl]-1H-indole-2-carboxamide,
N-(1-methylindazol-5-yl)-5-fluoro-1-[(3-fluorophenyl)methyl]-1H-indole-2-carboxamide,
N-(2-methylbenzoxazol-5-yl)-5-fluoro-1-[(3-fluorophenyl)methyl]-1H-indole-2-carboxamide,
N-(benzothiazol-6-yl)-5-fluoro-1-[(3-fluorophenyl)methyl]-1H-indole-2-carboxamide,
N-(2-methylbenzothiazol-5-yl)-5-fluoro-1-[(3-fluorophenyl)methyl]-1H-indole-2-carboxamide,
N-(2-oxo-3,4-dihydroquinol-7-yl)-5-trifluoromethyl-1-[(3-fluorophenyl)methyl]-1H-indole-2-carboxamide,
N-(2-oxoindolin-5-yl)-5-fluoro-1-[(3-fluorophenyl)methyl]-1H-indole-2-carboxamide,
N-(1H-benzotriazol-5-yl)-5-fluoro-1-[(3-fluorophenyl)methyl]-1H-indole-2-carboxamide,
N-(1-methylsulfonylindolin-5-yl)-5-fluoro-1-[(3-fluorophenyl)methyl]-1H-indole-2-carboxamide,
N-(1,2-dimethylbenzimidazol-5-yl)-5-fluoro-1-[(3-fluorophenyl)methyl]-1H-indole-2-carboxamide,
N-(2-ethylbenzoxazol-5-yl)-5-trifluoromethyl-1-[(3-fluorophenyl)methyl]-1H-indole-2-carboxamide,
N-(2-phenylbenzoxazol-5-yl)-5-trifluoromethyl-1-[(3-fluorophenyl)methyl]-1H-indole-2-carboxamide,
N-(quinoxalin-6-yl)-5-fluoro-1-[(3-fluorophenyl)methyl]-1H-indole-2-carboxamide,
N-(quinol-7-yl)-5-fluoro-1-[(3-fluorophenyl)methyl]-1H-indole-2-carboxamide,
N-(isoquinol-7-yl)-5-fluoro-1-[(3-fluorophenyl)methyl]-1H-indole-2-carboxamide,
N-(2-methylbenzimidazol-5-yl)-5-fluoro-1-[(3-fluorophenyl)methyl]-1H-indole-2-carboxamide,
N-(benzimidazol-5-yl)-5-fluoro-1-[(3-fluorophenyl)methyl]-1H-indole-2-carboxamide,
N-(2-oxo-3,4-dihydroquinol-7-yl)-6-methoxy-1-[(4-fluorophenyl)methyl]-1H-indole-2-carboxamide and
N-(1-methylbenzimidazol-6-yl)-5-fluoro-1-[(3-fluorophenyl)methyl]-1H-indole-2-carboxamide.

7. Process for preparing a compound of formula (I) according to any one of Claims 1 to 6, **characterized in that** a compound of general formula (IV)

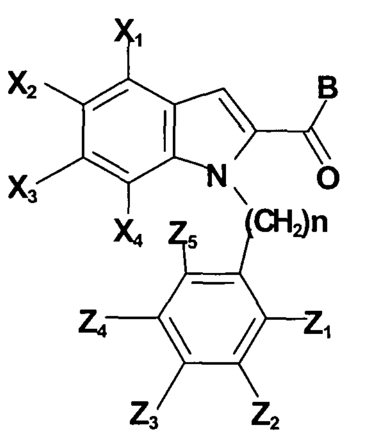

(IV)

in which $X_1$, $X_2$, $X_3$, $X_4$, $Z_1$, $Z_2$, $Z_3$, $Z_4$, $Z_5$ and n are as defined in the general formula (I) according to Claim 1 and B represents a $C_1$-$C_4$-alkoxy group,
is reacted with an amide of the compound of general formula (V)

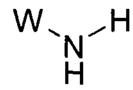

in which W is as defined in the general formula (I) according to Claim 1, at the reflux point of a solvent, the amide of the compound of general formula (V) being prepared via the prior action of trimethylaluminium on the compounds of general formula (V).

8. Process for preparing a compound of formula (I) according to any one of Claims 1 to 6, **characterized in that** a compound of general formula (IV)

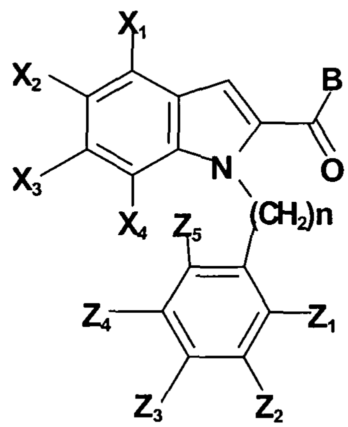

(IV)

in which $X_1$, $X_2$, $X_3$, $X_4$, $Z_1$, $Z_2$, $Z_3$, $Z_4$, $Z_5$ and n are as defined in the general formula (I) according to Claim 1 and B represents a hydroxyl group,
is converted into the acid chloride via the action of thionyl chloride at the reflux point of a solvent,
and the compound of general formula (IV) obtained, in which $X_1$, $X_2$, $X_3$, $X_4$, $Z_1$, $Z_2$, $Z_3$, $Z_4$, $Z_5$ and n are as defined in the general formula (I) according to Claim 1, and B represents a chlorine atom, is then reacted, in the presence of a base, with the compound of general formula (V)

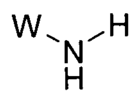

in which W is as defined in the general formula (I) according to Claim 1,
or alternatively a coupling reaction is performed between a compound of general formula (IV) in which $X_1$, $X_2$, $X_3$, $X_4$, $Z_1$, $Z_2$, $Z_3$, $Z_4$, $Z_5$ and n are as defined in the general formula (I) according to Claim 1 and B represents a hydroxyl group,
and the compound of general formula (V), in which W is as defined in the general formula (I) according to Claim 1, in the presence of a coupling agent and a base, in a solvent.

9. Medicament, **characterized in that** it comprises a compound of formula (I) according to any one of Claims 1 to 6, or a pharmaceutically acceptable salt, or a hydrate or a solvate of the compound of formula (I) .

10. Pharmaceutical composition, **characterized in that** it comprises a compound of formula (I) according to any one of Claims 1 to 6, or a pharmaceutically acceptable salt, a hydrate or a solvate of this compound, and also at least one pharmaceutically acceptable excipient.

11. Use of a compound of formula (I) according to any one of Claims 1 to 6, for the preparation of a medicament for preventing or treating pathologies in which the TRPV1 receptors are involved.

**12.** Use of a compound of formula (I) according to any one of Claims 1 to 6, for the preparation of a medicament for preventing or treating pain and inflammation, urological disorders, gynaecological disorders, gastrointestinal disorders, respiratory disorders, psoriasis, pruritus, dermal, ocular or mucous irritation, herpes and zona.

**13.** Use of a compound of formula (I) according to Claim 12, **characterized in that** pain and inflammation are chronic, neuropathic (trauma-related, diabetic, metabolic, infection-related or toxic, or induced by an anticancer or iatrogenic treatment), (osteo)arthritic, rheumatic in nature, or of the fibromyalgia, back pain, cancer-related pain, facial neuralgia, headaches, migraine, dental pain, burns, sunburn, animal bites or insect bites, post-herpetic neuralgia, muscular pain, trapped nerves (central and/or peripheral), spinal column and/or brain trauma, ischaemia (of the spinal column and/or the brain), neurodegeneration, haemorrhagic strokes (of the spinal column and/or of the brain) and post-stroke pain type.

**14.** Use of a compound of formula (I) according to Claim 12, **characterized in that** urological disorders are chosen from hyperactivity of the bladder, vesical hyperreflexia, vesical instability, incontinence, urgent micturition, urinary incontinence, cystitis, nephritic colic, pelvic hypersensitivity and pelvic pain.

**15.** Use of a compound of formula (I) according to Claim 12, **characterized in that** gynaecological disorders are chosen from vulvodynia and pain associated with salpingitis or with dysmenorrhoea.

**16.** Use of a compound of formula (I) according to Claim 12, **characterized in that** gastrointestinal disorders are chosen from gastrooesophageal reflux disorder, stomach ulcers, duodenal ulcers, functional dyspepsia, colitis, IBS, Crohn's disease, pancreatitis, oesophagitis and biliary colic.

**17.** Use of a compound of formula (I) according to Claim 12, **characterized in that** respiratory disorders are chosen from asthma, coughing, COPD, bronchoconstriction and inflammatory disorders.

**Patentansprüche**

**1.** Verbindung der Formel (I),

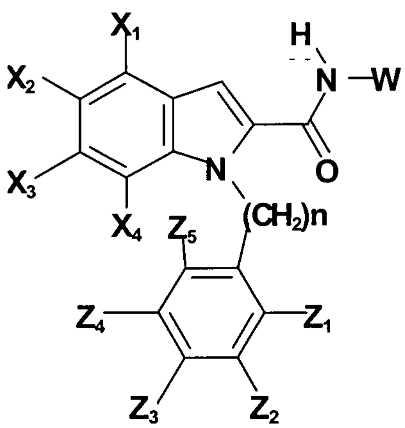

(I)

in der

n gleich 0, 1, 2 oder 3;

$X_1$, $X_2$, $X_3$, $X_4$, $Z_1$, $Z_2$, $Z_3$, $Z_4$ und $Z_5$ unabhängig voneinander ein Wasserstoff- oder Halogenatom oder eine $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, $C_1$-$C_6$-Alkoxy-, $C_1$-$C_6$-Fluoralkoxy-,Cyano-,C(O)$NR_1R_2$-,Nitro-,$NR_1R_2$-,$C_1$-$C_6$-Thioalkyl-,-S(O)-$C_1$-$C_6$-Alkyl-,-S(O)$_2$-$C_1$-$C_6$-Alkyl-, $SO_2NR_1R_2$-, $NR_3COR_4$-, $NR_3SO_2R_5$- oder Arylgruppe bedeuten, wobei das Aryl gegebenenfalls durch einen oder mehrere Substituenten aus der Reihe Halogen, $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, $C_1$-$C_6$-Alkoxy-, $C_1$-$C_6$-Fluor-alkoxy-, Nitro- oder Cyanogruppe substituiert ist;

$R_1$ und $R_2$ unabhängig voneinander ein Wasserstoffatom oder eine $C_1$-$C_6$-Alkyl-, $C_3$-$C_7$-Cycloalkyl-, $C_3$-$C_7$-Cycloalkyl-$C_1$-$C_3$-alkylen- oder Arylgruppe bedeuten; oder $R_1$ und $R_2$ zusammen mit dem Stickstoffatom, an das

sie gebunden sind, eine Azetidin-, Pyrrolidin-, Piperidin-, Azepin-, Morpholin-, Thiomorpholin-, Piperazin- oder Homopiperazingruppe bilden, wobei diese Gruppe gegebenenfalls durch eine $C_1$-$C_6$-Alkyl-, $C_3$-$C_7$-Cycloalkyl-, $C_3$-$C_7$-Cycloalkyl-$C_1$-$C_3$-alkylen- oder Arylgruppe substituiert ist;
$R_3$ und $R_4$ unabhängig voneinander ein Wasserstoffatom oder eine $C_1$-$C_6$-Alkyl- oder Arylgruppe bedeuten;
$R_5$ eine $C_1$-$C_6$-Alkyl- oder Arylgruppe bedeutet; W eine anellierte bicyclische Gruppe der Formel:

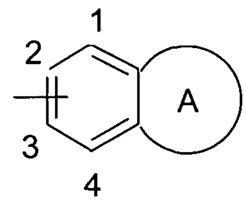

bedeutet, die über die 1-, 2-, 3- oder 4-Stellungen an das Stickstoffatom gebunden ist;
A einen Heterocyclus mit 5-7 Ringgliedern, der ein bis drei Heteroatome aus der Gruppe 0, S oder N umfaßt, bedeutet;
wobei das bzw. die Kohlenstoffatom(e) von A gegebenenfalls durch eine oder mehrere Gruppen aus der Reihe Wasserstoffatom oder $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, Aryl-, Aryl-$C_1$-$C_6$-alkylen-, Oxo-oder Thiogruppe substituiert ist/sind;
wobei das bzw. die Stickstoffatom(e) von A, wenn der Stickstoff einem durch eine Oxogruppe substituierten Kohlenstoffatom benachbart ist, durch $R_6$ substituiert ist/sind bzw. in den anderen Fällen durch $R_7$ substituiert ist/sind;
$R_6$ ein Wasserstoffatom oder eine $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, Aryl-$C_1$-$C_6$-alkylen- oder Arylgruppe bedeutet;
$R_7$ ein Wasserstoffatom oder eine $C_1$-$C_6$-Alkyl-, $C_3$-$C_7$-Ccycloalkyl-, $C_3$-$C_7$-Cycloalkyl-$C_1$-$C_3$-alkylen-, $C_1$-$C_6$-Fluoralkyl-, Aryl-$C_1$-$C_6$-alkylen-, $C_1$-$C_6$-Alkyl-C (O) -, $C_3$-$C_7$-Cycloalkyl-$C_1$-$C_3$-alkylen- (CO) -, $C_1$-$C_6$-Fluoralkyl-C (O) -, $C_3$-$C_7$-Cycloalkyl-C(O)-, Aryl-C(O)-, Aryl-$C_1$-$C_6$-alkylen-C(O)-, $C_1$-$C_6$-AlkylS$(O)_2$-, $C_1$-$C_6$-Fluoralkyl-S$(O)_2$-, $C_3$-$C_7$-Cycloalkyl-S$(O)_2$-, $C_3$-$C_7$-Cycloalkyl-$C_1$-$C_3$-alkylen-S$(O)_2$-, Aryl-S$(O)_2$- oder Aryl-$C_1$-$C_6$-alkylen-S$(O)_2$- oder Arylgruppe bedeutet; und
W nicht Indolyl bedeutet;
wobei das bzw. die Schwefelstoffatom(e) des Heterocyclus A in oxidierter Form vorliegen kann/können;
wobei das bzw. die Stickstoffatom(e) des Heterocyclus A in oxidierter Form vorliegen kann/können;
in Basenform oder in Säureadditionssalzform sowie in Hydratform oder Solvatform.

2. Verbindung der Formel (I) nach Anspruch 1, **dadurch gekennzeichnet, daß** n gleich 0 oder 1, in Basenform oder in Säureadditionssalzform sowie in Hydratform oder Solvatform.

3. Verbindung der Formel (I) nach Anspruch 1 oder 2, **dadurch gekennzeichnet, daß** $X_1$, $X_2$, $X_3$, $X_4$, $Z_1$, $Z_2$, $Z_3$, $Z_4$ und $Z_5$ unabhängig voneinander ein Wasserstoff- oder Halogenatom oder eine $C_1$-$C_6$-Alkyl-, $C_1$-$C_6$-Fluoralkyl- oder $C_1$-$C_6$-Alkoxygruppe bedeuten, in Basenform oder in Säureadditionssalzform sowie in Hydratform oder Solvatform.

4. Verbindung der Formel (I) nach einem der Ansprüche 1 bis 3, **dadurch gekennzeichnet, daß** W aus Indolinyl-, Isoindolinyl-, Benzofuranyl-, Dihydrobenzofuranyl-, Benzothiophenyl-, Dihydrobenzothiophenyl-, Benzoxazolyl-, Dihydrobenzoxazolinyl-, Isobenzofuranyl-, Dihydroisobenzofuranyl-, Benzimidazolyl-, Dihydrobenzimidazolyl-, Indazolyl-, Benzothiazolyl-, Isobenzothiazolyl-, Dihydroisobenzothiazolyl-, Benzotriazolyl-, Chinolinyl-, Dihydrochinolinyl-, Tetrahydrochinolinyl-, Isochinolinyl-, Dihydroisochinolinyl-, Tetrahydroisochinolinyl-, Benzoxazinyl-, Dihydrobenzoxazinyl-, Benzothiazinyl-, Dihydrobenzothiazinyl-, Cinnolinyl-, Chinazolinyl-, Dihydrochinazolinyl-, Tetrahydrochinazolinyl-, Chinoxalinyl-, Dihydrochinoxalinyl-, Tetrahydrochinoxalinyl-, Phthalazinyl-, Dihydrophthalazinyl-, Tetrahydrophthalazinyl-, Tetrahydrobenz[*b*]azepinyl-, Tetrahydrobenz[c]azepinyl-, Tetrahydrobenz[d]azepinyl-, Tetrahydrobenzo[*b*][1,4]diazepinyl-, Tetrahydrobenzo[*e*][1,4]diazepinyl-, Tetrahydrobenzo[*b*][1,4]oxazepinyl- oder Tetrahydrobenzo[b][1,4]thiazepinylgruppen stammt;
wobei das bzw. die Kohlenstoff- und/oder Stickstoffatom(e) der Gruppe W gegebenenfalls wie in der allgemeinen Formel (I) nach Anspruch 1 definiert substituiert ist/sind;
in Basenform oder in Säureadditionssalzform sowie in Hydratform oder Solvatform.

5. Verbindung der Formel (I) nach Anspruch 4, **dadurch gekennzeichnet, daß** W aus Isochinolinyl-, Dihydrochinolinyl-,

Tetrahydrochinolinyl-, Benzoxazinyl-, Dihydrobenzoxazinyl-, Benzofuranyl-, Indolinyl-, Benzoxazolyl-, Indazolyl-, Benzimidazolyl-, Benzothiazolyl-, Benzotriazolyl-, Chinolinyl-, Chinoxalinylgruppen stammt;
wobei das bzw. die Kohlenstoffatom(e) der Gruppe W gegebenenfalls durch eine oder mehrere Gruppen aus der Reihe Oxo-, $C_1$-$C_6$-Alkyl-, oder Arylgruppe wie in der allgemeinen Formel (I) in Bezug auf A definiert substituiert ist/sind; und/oder
wobei das oder die Stickstoffatom(e) der Gruppe W, wenn der Stickstoff einem durch eine Oxogruppe substituierten Kohlenstoffatom benachbart ist, durch $R_6$ substituiert ist/sind bzw. in den anderen Fällen durch $R_7$ substituiert ist/sind; wobei $R_6$ und $R_7$ wie in der allgemeinen Formel (I) nach Anspruch 1 in Bezug auf A definiert sind,
wobei $R_6$ ein Wasserstoffatom oder eine $C_1$-$C_6$-Alkylgruppe bedeutet,
wobei $R_7$ ein Wasserstoffatom oder eine $C_1$-$C_6$-Alkyl-oder $C_1$-$C_6$-Alkyl-S(O)$_2$-Gruppe bedeutet;
in Basenform oder in Säureadditionssalzform sowie in Hydratform oder Solvatform.

**6.** Verbindung der Formel (I) nach Anspruch 1, ausgewählt aus:

*N*-(Isochinolin-5-yl)-5-fluor-1-[((3-trifluormethyl)phenyl)methyl]-1*H*-indol-2-carboxamid,
*N*-(1-Methyl-1,2,3,4-tetrahydrochinolin-7-yl)-1-[3-(trifluormethyl)phenyl]-1*H*-indol-2-carboxamid,
*N*-(1-Methyl-1,2,3,4-tetrahydrochinolin-7-yl)-1-((3-5-dimethyl)phenyl)-1*H*-indol-2-carboxamid,
*N*-(1,2,3,4-Tetrahydrochinolin-7-yl)-1-[3-(trifluormethyl)phenyl]-1*H*-indol-2-carboxamid,
*N*-(4-Methyl-3-oxo-2*H*-benzoxazin-7-yl)-1-[3-(trifluormethyl)phenyl]-1*H*-indol-2-carboxamid,
*N*-(4-Methyl-3-oxo-2*H*-benzoxazin-6-yl)-1-[3-(trifluormethyl)phenyl]-1*H*-indol-2-carboxamid,
*N*-(2-Oxo-3,4-dihydrochinolin-7-yl)-1-[3-(trifluormethyl)phenyl]-1*H*-indol-2-carboxamid,
*N*-(Benzofuran-5-yl)-1-[3-(trifluormethyl)phenyl]-1*H*-indol-2-carboxamid,
*N*-(1-Methylindolin-5-yl)-1-[3-(trifluormethyl)phenyl]-1*H*-indol-2-carboxamid,
*N*-(2,3-Dihydrobenzoxazin-6-yl)-1-[3-(trifluormethyl)phenyl]-1*H*-indol-2-carboxamid,
*N*-(3-Oxo-2*H*-benzoxazin-7-yl)-1-[3-(trifluormethyl)phenyl]-1*H*-indol-2-carboxamid,
*N*-(1-Methylindolin-5-yl)-5-fluor-1-[(3-fluorphenyl)methyl]-1*H*-indol-2-carboxamid,
*N*-(1-Methyl-1,2,3,4-tetrahydrochinolin-7-yl)-5-fluor-1-[(3-fluorphenyl)methyl]-1*H*-indol-2-carboxamid,
*N*-(1,2,3,4-Tetrahydrochinolin-7-yl)-5-fluor-1-[(3-fluorphenyl)methyl]-1*H*-indol-2-carboxamid,
*N*-(Isochinolin-5-yl)-5-fluor-1-[(3-fluorphenyl)methyl]-1*H*-indol-2-carboxamid,
*N*-(1,2,3,4-Tetrahydrochinolin-8-yl)-1-[3-(trifluormethyl)phenyl]-1*H*-indol-2-carboxamid,
*N*-(Benzoxazol-5-yl)-1-[3-(trifluormethyl)phenyl]-1*H*-indol-2-carboxamid,
*N*-(2-Methylbenzoxazol-5-yl)-1-[3-(trifluormethyl)phenyl]-1*H*-indol-2-carboxamid,
*N*-(1-Methyl-1*H*-indazol-5-yl)-5-trifluormethyl-1-[(3-fluorphenyl)methyl]-1*H*-indol-2-carboxamid,
*N*-(2-Oxo-3,4-dihydrochinolin-7-yl)-5-fluor-1-[(3-fluorphenyl)methyl]-1*H*-indol-2-carboxamid,
*N*-(Benzofuran-5-yl)-5-fluor-1-[(3-fluorphenyl)methyl]-1*H*-indol-2-carboxamid,
*N*-(2,3-Dihydrobenzoxazin-6-yl)-5-fluor-1-[(3-fluorphenyl)methyl]-1*H*-indol-2-carboxamid,
*N*-(3-Oxo-2*H*-benzoxazin-6-yl)-5-fluor-1-[(3-fluorphenyl)methyl]-1*H*-indol-2-carboxamid,
*N*-(1,2,3,4-Tetrahydrochinolin-7-yl)-5-trifluormethyl-1-[(3-fluorphenyl)methyl]-1*H*-indol-2-carboxamid,
*N*-(2-Oxoindolin-5-yl)-5-trifluormethyl-1-[(3-fluorphenyl)methyl]-1*H*-indol-2-carboxamid,
*N*-(1-Methylbenzimidazol-5-yl)-5-trifluormethyl-1-[(3-fluorphenyl)methyl]-1*H*-indol-2-carboxamid,
*N*-(1-Methyl-1,2,3,4-tetrahydrochinolin-7-yl)-5-trifluormethyl-1-[(3-fluorphenyl)methyl]-1*H*-indol-2-carboxamid,
*N*-(Benzothiazol-6-yl)-5-trifluormethyl-1-[(3-fluorphenyl)methyl]-1*H*-indol-2-carboxamid,
*N*-(2-Methylbenzoxazol-5-yl)-5-trifluormethyl-1-[(3-fluorphenyl)methyl]-1*H*-indol-2-carboxamid,
*N*-(2-Methylbenzothiazol-5-yl)-5-trifluormethyl-1-[(3-fluorphenyl)methyl]-1*H*-indol-2-carboxamid,
*N*-(1-Methylsulfonylindolin-5-yl)-5-trifluormethyl-1-[(3-fluorphenyl)methyl]-1*H*-indol-2-carboxamid,
*N*-(Isochinolin-6-yl)-5-fluor-1-[(3-fluorphenyl)methyl]-1*H*-indol-2-carboxamid,
*N*-(1-Methylbenzimidazol-5-yl)-5-fluor-1-[(3-fluorphenyl)methyl]-1*H*-indol-2-carboxamid,
*N*-(1-Methylbenzimidazol-4-yl)-5-fluor-1-[(3-fluorphenyl)methyl]-1*H*-indol-2-carboxamid,
*N*-(1*H*-Benzotriazol-5-yl)-5-trifluormethyl-1-[(3-fluorphenyl)methyl]-1*H*-indol-2-carboxamid,
*N*-(Chinolin-6-yl)-5-fluor-1-[(3-fluorphenyl)methyl]-1*H*-indol-2-carboxamid,
*N*-(1-Methylindazol-5-yl)-5-fluor-1-[(3-fluorphenyl)methyl]-1*H*-indol-2-carboxamid,
*N*-(2-Methylbenzoxazol-5-yl)-5-fluor-1-[(3-fluorphenyl)methyl]-1*H*-indol-2-carboxamid,
*N*-(Benzothiazol-6-yl)-5-fluor-1-[(3-fluorphenyl)methyl]-1*H*-indol-2-carboxamid,
*N*-(2-Methylbenzothiazol-5-yl)-5-fluor-1-[(3-fluorphenyl)methyl]-1*H*-indol-2-carboxamid,
*N*-(2-Oxo-3,4-dihydrochinolin-7-yl)-5-trifluormethyl-1-[(3-fluorphenyl)methyl]-1*H*-indol-2-carboxamid,
*N*-(2-Oxoindolin-5-yl)-5-fluor-1-[(3-fluorphenyl)methyl]-1*H*-indol-2-carboxamid,
*N*-(1*H*-Benzotriazol-5-yl)-5-fluor-1-[(3-fluorphenyl)methyl]-1*H*-indol-2-carboxamid,
*N*-(1-Methylsulfonylindolin-5-yl)-5-fluor-1-[(3-fluorphenyl)methyl]-1*H*-indol-2-carboxamid,

*N*-(1,2-Dimethylbenzimidazol-5-yl)-5-fluor-1-[(3-fluorphenyl)methyl]-1*H*-indol-2-carboxamid,
*N*-(2-Ethylbenzoxazol-5-yl)-5-trifluormethyl-1-[(3-fluorphenyl)methyl]-1*H*-indol-2-carboxamid,
*N*-(2-Phenylbenzoxazol-5-yl)-5-trifluormethyl-1-*[*(3-fluorphenyl)methyl]-1*H*-indol-2-carboxamid,
*N*-(Chinoxalin-6-yl)-5-fluor-1-[(3-fluorphenyl)methyl]-1*H*-indol-2-carboxamid,
*N*-(Chinolin-7-yl)-5-fluor-1-[(3-fluorphenyl)methyl]-1*H*-indol-2-carboxamid,
*N*-(Isochinolin-7-yl)-5-fluor-1-[(3-fluorphenyl)methyl]-1*H*-indol-2-carboxamid,
*N*-(2-Methylbenzimidazol-5-yl)-5-fluor-1-[(3-fluorphenyl)methyl]-1*H*-indol-2-carboxamid,
*N*-(Benzimidazol-5-yl)-5-fluor-1-[(3-fluorphenyl)methyl]-1*H*-indol-2-carboxamid,
*N*-(2-Oxo-3,4-dihydrochinolin-7-yl)-6-methoxy-1-[(4-fluorphenyl)methyl]-1*H*-indol-2-carboxamid und
*N*-(1-Methylbenzimidazol-6-yl)-5-fluor-1-[(3-fluorphenyl)methyl]-1*H*-indol-2-carboxamid.

7. Verfahren zur Herstellung einer Verbindung der Formel (I) nach einem der Ansprüche 1 bis 6, **dadurch gekennzeichnet, daß** man eine Verbindung der allgemeinen Formel (IV),

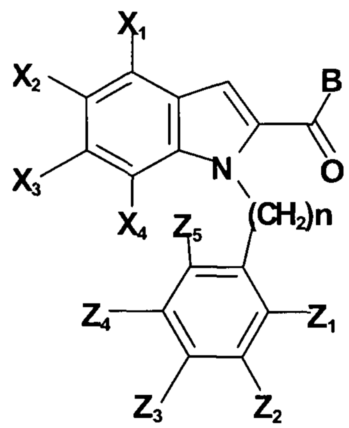

(IV)

in der $X_1$, $X_2$, $X_3$, $X_4$, $Z_1$, $Z_2$, $Z_3$, $Z_4$, $Z_5$ und n wie in der allgemeinen Formel (I) nach Anspruch 1 definiert sind und B eine $C_1$-$C_4$-Alkoxygruppe bedeutet,
mit einem Amid der Verbindung der allgemeinen Formel (V),

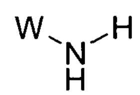

in der W wie in der allgemeinen Formel (I) nach Anspruch 1 definiert ist, unter Rückfluß eines Lösungsmittels umsetzt, wobei das Amid der Verbindung der allgemeinen Formel (V) durch zuvoriges Einwirken von Trimethylaluminium auf die Verbindungen der allgemeinen Formel (V) hergestellt worden ist.

8. Verfahren zur Herstellung einer Verbindung der Formel (I) nach einem der Ansprüche 1 bis 6, **dadurch gekennzeichnet, daß** man eine Verbindung der allgemeinen Formel (IV)

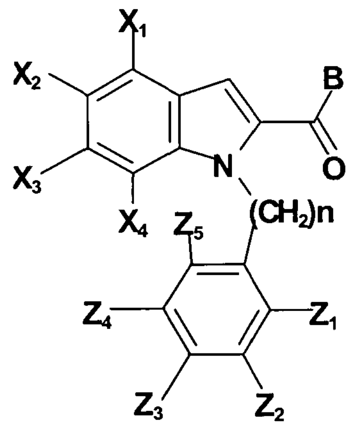

(IV)

in der $X_1$, $X_2$, $X_3$, $X_4$, $Z_1$, $Z_2$, $Z_3$, $Z_4$, $Z_5$ und n wie in der allgemeinen Formel (I) nach Anspruch 1 definiert sind und B eine Hydroxylgruppe bedeutet, durch Einwirken von Thionylchlorid unter Rückfluß eines Lösungsmittels in das Säurechlorid umwandelt und das man dann die erhaltene Verbindung der allgemeinen Formel (IV), in der $X_1$, $X_2$, $X_3$, $X_4$, $Z_1$, $Z_2$, $Z_3$, $Z_4$, $Z_5$ und n wie in der allgemeinen Formel (I) nach Anspruch 1 definiert sind und B ein Chloratom bedeutet, mit der Verbindung der allgemeinen Formel (V),

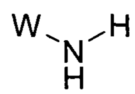

in der W wie in der allgemeinen Formel (I) nach Anspruch 1 definiert ist oder **dadurch**, daß man zwischen einer Verbindung der allgemeinen Formel (IV), in der $X_1$, $X_2$, $X_3$, $X_4$, $Z_1$, $Z_2$, $Z_3$, $Z_4$, $Z_5$ und n wie in der allgemeinen Formel (I) nach Anspruch 1 definiert sind und B eine Hydroxylgruppe bedeutet und der Verbindung der allgemeinen Formel (V), in der W wie in der allgemeinen Formel (I) nach Anspruch 1 definiert ist, in Gegenwart eines Kupplungsmittels und einer Base in einem Lösungsmittel eine Kopplungsreaktion durchführt.

**9.** Arzneimittel, **dadurch gekennzeichnet, daß** es eine Verbindung der Formel (I) nach einem der Ansprüche 1 bis 6 oder ein pharmazeutisch unbedenkliches Salz oder auch ein Hydrat oder Solvat der Verbindung der Formel (I) umfaßt.

**10.** Pharmazeutische Zusammensetzung, **dadurch gekennzeichnet, daß** sie eine Verbindung der Formel (I) nach einem der Ansprüche 1 bis 6 oder ein pharmazeutisch unbedenkliches Salz, ein Hydrat oder ein Solvat dieser Verbindung sowie mindestens einen pharmazeutisch unbedenklichen Träger umfaßt.

**11.** Verwendung einer Verbindung der Formel (I) nach einem der Ansprüche 1 bis 6 zur Herstellung eines Arzneimittels für die Vorbeugung oder Behandlung von Pathologien, an denen die Rezeptoren des TRPVI-Typs beteiligt sind.

**12.** Verwendung einer Verbindung der Formel (I) nach einem der Ansprüche 1 bis 6 zur Herstellung eines Arzneimittels für die Vorbeugung oder Behandlung von Schmerz und Entzündung, Störungen der Harnwege, gynäkologische Störungen, Störungen des Magen-Darm-Trakts, Störungen der Atemwege, Psoriasis, Pruritus, Haut-, Augen- oder Schleimhautentzündungen, Herpes und Gürtelrose.

**13.** Verwendung einer Verbindung der Formel (I) nach Anspruch 12, **dadurch gekennzeichnet, daß** der Schmerz und die Entzündung chronischer Natur, neuropathischer Natur (traumatisch, diabetisch, metabolisch, infektiös, toxisch, durch Antikrebsbehandlung induziert oder iatrogen), (osteo)arthritischer Natur, rheumatischer Natur oder vom Typ Fibromyalgien, Rückenschmerzen, mit Krebs im Zusammenhang stehende Schmerzen, Gesichtsneuralgie, Kopfneuralgie, Migräne, Zahnschmerzen, Verbrennung, Sonnenstich, Bisse oder Stiche, Post-Herpes-Neuralgie, Muskelschmerzen, Nervenkompression (zentral und/oder peripher), Traumen des Rückenmarks und/oder des Hirns, Ischämie (des Rückenmarks und/oder des Hirns), Neurodegeneration, hämorrhagische Gefäßkomplikationen (des Rückenmarks und/oder des Hirns) Schmerzen nach Schlaganfall sind.

**14.** Verwendung einer Verbindung der Formel (I) nach Anspruch 12, **dadurch gekennzeichnet, daß** die Störungen der Harnwege aus der Reihe hyperaktive Harnblase, Blasenhyperreflexie, instabile Harnblase, Inkontinenz, Harndrang, Harninkontinenz, Cystitis, Nierenkolik, Hypersensitivität des Beckens und Beckenschmerzen stammen.

**15.** Verwendung einer Verbindung der Formel (I) nach Anspruch 12, **dadurch gekennzeichnet, daß** die gynäkologischen Störungen aus der Reihe Vulvodynie, salpingitisassoziierte Schmerzen und Dysmenorrhö stammen.

**16.** Verwendung einer Verbindung der Formel (I) nach Anspruch 12, **dadurch gekennzeichnet, daß** die Magen-Darm-Störungen aus der Reihe Störung des Magen-Schlund-Reflexes, Magengeschwür, Zwölffingerdarmgeschwür, funktionelle Dyspepsie, Colitis, Reizdarm-Syndrom, Morbus Crohn, Pankreatitis, Ösophagitis und Leberkolik stammen.

**17.** Verwendung einer Verbindung der Formel (I) nach Anspruch 12, **dadurch gekennzeichnet, daß** die Störungen der Atemwege aus der Reihe Asthma, Husten, COPD, Bronchuskonstriktion und entzündliche Störungen stammen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 20050165049 A
- JP 2001151771 A
- WO 2003049702 A
- WO 2003068749 A


### Littérature non-brevet citée dans la description


- **J. March.** Advances in Organic Chemistry. Wiley Interscience, 2001
- **Kolasa T.** *Bioorg.Med.Chem.,* 1997, vol. 5 (3), 507
- **Abramovitch R.** *Synth. Commun.,* 1995, vol. 25 (1), 1
- **Murakami Y.** *Chem.Pharm.Bull.,* 1995, vol. 43 (8), 1281
- **S.L. Buchwald.** *J.Am.Chem.Soc.,* 2002, vol. 124, 11684
- **W.W.K.R. Mederski.** *Tetrahedron,* 1999, vol. 55, 12757
- **D. Knittel.** *Synthesis,* 1985, vol. 2, 186
- **T.M. Williams.** *J.Med.Chem.,* 1993, vol. 36 (9), 1291
- *Pharmazie,* 1990, vol. 45, 346
- **I.T. Forbes.** *J. Med. Chem.,* 1993, vol. 36 (8), 1104